# EUROPÄISCHE PATENTANMELDUNG

(11) **EP 4 570 159 A1**
(43) Veröffentlichungstag der Anmeldung: **18.06.2025**
(21) Anmeldenummer: 24219273.0
(22) Anmeldetag: 12.12.2024
(51) Int. Cl.: A61B 1/00, A61B 1/05, A61B 5/00, G02B 23/24, H04N 23/50, G01J 3/28

(54) **ENDOSKOPVORRICHTUNG UND MEDIZINISCHES SYSTEM ZUR MEDIZINISCHEN BILDGEBUNG**

(30) Priorität: 13.12.2023 DE 102023134893
(71) Anmelder: Karl Storz SE & Co. KG, 78532 Tuttlingen (DE)
(72) Erfinder: FORSTER, Jonas, 78532 Tuttlingen (DE)

(57) **Zusammenfassung**

Die Erfindung betrifft Endoskopvorrichtung (10) mit einem Schaft (12), der einen proximalen und einen distalen Abschnitt (14, 16) aufweist. Ferner umfasst die Endoskopvorrichtung (10) eine hyperspektrale Bilderfassungsbaugruppe (18), die dazu eingerichtet ist, Bilder eines Objektbereichs (20) zu erfassen und hyperspektrale Bilddaten zu erzeugen, die räumliche und spektrale information umfassen. Die Bilderfassungsbaugruppe (18) ist in dem distalen Abschnitt (16) des Schafts (12) angeordnet.

Ferner betrifft die Erfindung ein Endoskop (70) und ein medizinisches System (72).

## Beschreibung

Die Erfindung betrifft eine Endoskopvorrichtung, ein Endoskop sowie ein medizinisches System zur medizinischen Bildgebung.

Aus dem Stand der Technik sind endoskopische Vorrichtungen bekannt, die Multispektral- oder Hyperspektralbilder erzeugen. Multispektral- oder Hyperspektralbilder weisen neben zwei räumlichen Dimensionen, wie sie etwa ein herkömmliches Bild einer Kamera hat, eine spektrale Dimension auf. Die spektrale Dimension umfasst mehrere Spektralbänder (Wellenlängenbänder). Multispektrale und hyperspektrale Bilder unterscheiden sich im Wesentlichen in der Anzahl und/oder der Breite ihrer spektralen Bänder.

Es sind einige Bildgebungsvorrichtungen zur Erzeugung solcher Multispektral- oder Hyperspektralbilder, insbesondere im Kontext medizinischer Anwendungen, bekannt. In DE 20 2014 010 558 U1 ist beispielsweise eine Vorrichtung zur Aufnahme eines Hyperspektralbilds eines Untersuchungsgebiets eines Objekts beschrieben. In der Vorrichtung sind ein Eingangsobjektiv zur Erzeugung eines Bilds in einer Bildebene sowie eine schlitzförmige Blende in der Bildebene zur Auswahl eines schlitzförmigen Bereichs des Bilds angeordnet. Das durch die Blende hindurchtretende Licht wird mittels eines dispersiven Elements aufgefächert und mittels eines Bildsensors aufgenommen. Dadurch kann von dem Bildsensor eine Vielzahl von Spektren mit jeweils zugeordneter räumlicher Koordinate entlang der Längsrichtung der schlitzförmigen Blende aufgenommen werden. Die beschriebene Vorrichtung ist weiterhin dazu eingerichtet, in einer von der Längsrichtung der schlitzförmigen Blende verschiedenen Richtung weitere Spektren entlang der Längsrichtung der schlitzförmigen Blende aufzunehmen. Entweder durch sukzessives Verschieben des zu untersuchenden Objekts oder durch eine schrittweise Bewegung der Aufnahmevorrichtung über dem zu untersuchenden Körper wird eine translatorische Relativbewegung des Untersuchungsgebiets eines Körpers gegenüber dem Kamerasensor erzeugt. Auf diese Weise können aufeinanderfolgende schlitzförmige Teilabschnitte des Untersuchungsgebiets mit Spektren und Ortskoordinaten aufgenommen und mittels einer Datenverarbeitungseinrichtung zu einem Gesamtbild zusammengesetzt werden. Das dieser Offenbarung zugrunde liegende Verfahren zur Erzeugung von Multispektral- oder Hyperspektralbildern ist auch als sogenanntes Pushbroom-Verfahren bekannt.

Neben dem Pushbroom-Verfahren sind weitere Technologien zur Generierung multispektraler und/oder hyperspektraler Bilddaten bekannt. Beispielsweise wird beim sogenannten Whiskbroom-Ansatz das abzubildende Objekt punktweise abgefahren, wobei für jeden Punkt ein Spektrum gewonnen wird. Daneben ist es auch möglich, mehrere Bilder durch unterschiedliche Spektralfilter hindurch sequenziell in einem sogenannten Staring-Ansatz aufzunehmen. Jedes einzelne aufgenommene Bild enthält dabei vollständige räumliche Information für einen einzelnen Spektralbereich. Eine weitere Technologie zur Erfassung multispektraler und/oder hyperspektraler Bilddaten stellt das Snapshot-Verfahren dar. Mittels geeigneter optischer Elemente wie optischen Slicern, Linsen und Prismen wird ein zweidimensionales Mehrfarbenbild in mehrere spektrale Einzelbilder und/oder Bildteilbereiche zerlegt, die gleichzeitig auf unterschiedlichen Detektoren oder Detektorbereichen erfasst werden. Mit diesem Verfahren können spektrale Daten für ein gesamtes Bildfeld gleichzeitig in einem einzigen Erfassungsschritt aufgenommen werden.

Insbesondere im Zusammenhang mit minimalinvasiven chirurgischen Verfahren, aber auch generell zur Diagnostik sowie zur Beurteilung eines Erfolgs bzw. einer Qualität eines Eingriffs sind bildgebende Verfahren im medizinischen Bereich von zentraler Bedeutung. Beispielsweise kann ein Situs bei einem minimalinvasiven Eingriff beobachtet werden, wenn Gewebe verschweißt werden soll oder verschweißt wurde, etwa unter Verwendung eines elektrochirurgischen Geräts wie eines Hochfrequenz- Koagulationsinstruments. Weitere Beispiele für diagnostische und/oder therapeutische Aktionen, vor, während oder nach denen eine Beobachtung mittels bildgebender Verfahren zweckmäßig ist, sind die Beobachtung und Beurteilung von Ligaturen, Clips und Stapler-Einsätzen, eine Gewebeprobenentnahme oder generell verschiedene chirurgische Eingriffe. Für die meisten chirurgischen Verfahren ist es vorteilhaft, spektrale Bilddaten in Echtzeit zu erzeugen, sodass diese beispielsweise zur Überwachung und/oder zur Beurteilung einer diagnostischen und/oder therapeutischen Aktion genutzt werden können. Dies beinhaltet beispielsweise die Erzeugung eines spektral aufgelösten Bildes in weniger als einer Sekunde oder sogar mehrmals pro Sekunde.

Die bereits voranstehend erwähnten Verfahren zum Erzeugen hyperspektraler Bilddaten sind aufgrund ihrer Funktionsprinzipien prozesstechnisch sehr aufwendig. Zur Erzeugung eines Hyperspektralbilds eines Untersuchungsgebiets eines Objekts mittels des Pushbroom- sowie des Whiskbroom-Verfahrens, ist eine hochpräzise Relativbewegung des abzubildenden Objekts gegenüber dem Sensor zwingend erforderlich. Eine solche Relativbewegung wird üblicherweise erzeugt, indem der Sensor relativ zum abzubildenden Objekt bewegt wird. Ein hochgenaues Abscannen eines Untersuchungsgebiets nach einer dieser beiden Methoden erfordert also einen komplexen Aufbau der Bildgebungsvorrichtung mit einem akkuraten Aktuator, der nicht nur Raum einnimmt, sondern auch einen relevanten Kostenfaktor hinsichtlich der Anschaffungs- und Wartungskosten darstellt. Aufgrund der Größe herkömmlicher Kameras zur hyperspektralen Bildgebung, werden jene Kameras bislang außerhalb eines Endoskopschafts verbaut, sodass Licht mit Hilfe geeigneter optischer Elemente, beispielsweise mittels Stablinsen, über lange Strecken bis hin zum Kamerasensor geführt werden muss. Der Erfinder hat erkannt, dass derartige optische Elemente beispielsweise Absorptions-, Dispersions und/oder Streueffekte verursachen und damit vor allem die Qualität der Bilddaten maßgeblich beeinträchtigen. Ferner hat der Erfinder erkannt, dass aufgrund der proximalen Anordnung der Hyperspektralkamera starre Endoskopschäfte verwendet werden müssen, um das Abbildungslicht mittels der erwähnten optischen Elemente zuverlässig derart zum Kamerasensor zu führen, dass eine verwertbare Abbildung erzeugbar ist.

Ferner nimmt bei dem Pushbroom- oder Whiskbroom- und auch Staring-Ansatz die Erzeugung von Bilddaten viel Zeit in Anspruch. Eine Echtzeitüberwachung einer diagnostischen und/oder therapeutischen Aktion mit einem dieser drei Verfahren ist damit nur eingeschränkt möglich. Mittels des Snapshot-Verfahrens ist im Gegensatz zu den anderen, seriellen Systemen zwar eine sehr viel schnellere Datenerfassung möglich, jedoch weist diese Technologie, durch ihren Aufbau bedingt, nur eine sehr begrenzte räumliche Auflösung auf, wodurch die visuelle Bildqualität beeinträchtigt, und eine Analyse der Bilder erschwert wird.

Der Erfindung liegt insbesondere die Aufgabe zugrunde, die Einsatzmöglichkeiten hyperspektraler Bildgebung zu erweitern.

Diese Aufgabe wird erfindungsgemäß durch die Merkmale der unabhängigen Ansprüche gelöst. Weiterbildungen der Erfindung sind den abhängigen Ansprüchen zu entnehmen.

Eine erfindungsgemäße Endoskopvorrichtung umfasst einen Schaft, der einen proximalen und einen distalen Abschnitt aufweist. Ferner umfasst die Endoskopvorrichtung eine hyperspektrale Bilderfassungsbaugruppe, die dazu eingerichtet ist, Bilder eines während eines therapeutischen und/oder diagnostischen Verfahrens relevanten Objektbereichs zu erfassen und hyperspektrale Bilddaten zu erzeugen, die räumliche und spektrale Information umfassen. Die Bilderfassungsbaugruppe ist in dem distalen Abschnitt des Schafts angeordnet.

Durch die erfindungsgemäßen Merkmale können die Einsatzmöglichkeiten hyperspektraler Bildgebung erweitert werden. Diese Merkmale gestatten es zudem, eine Endoskopvorrichtung mit einer kompakten Bauweise sowie kurzen Lichtwegen einzusetzen. Insbesondere ermöglicht es die Erfindung, HSI-Bildgebung durchzuführen und zugleich die Vorteile auszunutzen, welche die Anordnung einer Kamera im distalen Schaftabschnitt eines Endoskops mit sich bringt. Ferner lässt sich mit einer erfindungsgemäßen Endoskopvorrichtung bei der Durchführung und/oder Beurteilung diagnostischer und/oder therapeutischer Aktionen ein hoher Grad an Qualität erzielen.

Unter einer "Endoskopvorrichtung" soll insbesondere ein, vorzugsweise funktionsfähiger, Bestandteil eines Endoskops, insbesondere eine Unterbaugruppe und/oder eine Konstruktions- und/oder eine Funktionskomponente eines Endoskops, verstanden werden. Die Endoskopvorrichtung ist beispielsweise dazu eingerichtet, zumindest teilweise und vorzugsweise zumindest zu einem Großteil in eine künstliche oder natürliche Öffnung, insbesondere Körperöffnung, eingeführt zu werden, um dort eine Behandlung oder Untersuchung vorzunehmen. Unter "eingerichtet" soll insbesondere speziell programmiert, vorgesehen, ausgelegt, ausgebildet und/oder ausgestattet verstanden werden. Darunter, dass ein Objekt zu einer bestimmten Funktion eingerichtet ist, soll ferner verstanden werden, dass das Objekt diese bestimmte Funktion in zumindest einem Anwendungs- oder Betriebszustand erfüllt oder ausführt.

Die Endoskopvorrichtung ist zur medizinischen Bildgebung eingerichtet. Unter einer "medizinischen Bildgebung" soll insbesondere eine Bildgebung verstanden werden, welche Rückschlüsse auf physiologische Eigenschaften eines abzubildenden Objektbereichs zulässt, wie beispielsweise eine Anatomie, einen Fettgehalt, einen Wassergehalt, eine Oxygenierung, ein Vorhandensein eines Farbstoffs oder dergleichen. Bei dem Objektbereich handelt es sich insbesondere um einen Bereich, der physiologische Bestandteile umfasst, wie beispielsweise Gewebe, Blut oder dergleichen. Der Objektbereich kann ferner ein Bildbereich sein, der wenigstens einen Teil und/oder Abschnitt eines abgebildeten Objekts umfasst. Der Objektbereich kann Gewebe und/oder Organe und/oder einen Teil eines Körpers eines Patienten betreffen. Beispielsweise kann das Objekt ein Situs sein. Der Objektbereich kann einen Situs betreffen. Der Objektbereich liegt beispielsweise innerhalb einer natürlichen oder künstlich geschaffenen Kavität. Solche Kavitäten sind in etwa der Bauchraum, der Darm, die Blase, die Niere oder dergleichen. Allerdings könnte auch offenes Gewebe als Objektbereich dienen. Die Endoskopvorrichtung ist in einigen Ausführungsformen dazu eingerichtet, zur Begutachtung und/oder Beobachtung zumindest abschnittsweise in einen Hohlraum einführbar zu sein, beispielsweise in eine künstliche und/oder natürliche Kavität, etwa in ein Inneres eines Körpers, in ein Körperorgan, in Gewebe oder dergleichen. Die Endoskopvorrichtung kann auch dazu eingerichtet sein, zur Begutachtung und/oder Beobachtung in ein Gehäuse, eine Verschalung, einen Schacht, ein Rohr oder eine andere, insbesondere künstliche, Struktur einführbar zu sein.

Die Endoskopvorrichtung weist insbesondere einen Schaft auf, innerhalb dessen die hyperspektrale Bilderfassungsbaugruppe angeordnet ist. Der Schaft ist insbesondere dazu eingerichtet, wenigstens teilweise und vorzugsweise wenigstens zu einem Großteil in eine insbesondere künstliche oder natürliche Öffnung, insbesondere Körperöffnung, eingeführt zu werden. Unter "distal" soll insbesondere bei einer Bedienung einem Patienten bzw. einem Objektbereich zugewandt und einem Bediener abgewandt verstanden werden. Unter "proximal" soll insbesondere bei einer Bedienung einem Patienten bzw. einem Objektbereich abgewandt und einem Bediener zugewandt verstanden werden. Der Schaft ist als längliches Bauteil ausgebildet. Unter einem "länglichen Bauteil" soll insbesondere ein Bauteil verstanden werden, dessen Haupterstreckung zumindest um einen Faktor fünf, vorzugsweise um einen Faktor zehn und besonders bevorzugt um einen Faktor zwanzig größer ist als eine Erstreckung des Bauteils zumindest im Wesentlichen senkrecht zur Haupterstreckung, also insbesondere einem Durchmesser des Bauteils. Unter einer "Haupterstreckungsrichtung" eines Bauteils soll dabei insbesondere eine Richtung verstanden werden, die parallel zu einer längsten Kante eines kleinsten gedachten Quaders verläuft, der das Bauteil gerade noch vollständig umschließt. Unter "zumindest". Unter "zumindest im Wesentlichen parallel" soll hier insbesondere eine Ausrichtung einer Richtung relativ zu einer Bezugsrichtung, insbesondere eine Ausrichtung einer Richtung relativ zu einer Bezugsrichtung, insbesondere in einer Ebene, verstanden werden, wobei die Richtung und die Bezugsrichtung einen Winkel von 0° insbesondere unter Berücksichtigung einer maximalen Abweichung von kleiner als 8°, vorteilhaft von kleiner als 5° und besonders vorteilhaft von kleiner als 2° einschließt. Unter "zumindest im Wesentlichen senkrecht" soll hier insbesondere eine Ausrichtung einer Richtung relativ zu einer Bezugsrichtung, insbesondere in einer Ebene, verstanden werden, wobei die Richtung und die Bezugsrichtung einen Winkel von 90° insbesondere unter Berücksichtigung einer maximalen Abweichung von kleiner als 8°, vorteilhaft von kleiner als 5° und besonders vorteilhaft von kleiner als 2° einschließt. Unter einer "Haupterstreckung" eines Objekts soll dabei insbesondere eine Erstreckung des Objekts verstanden werden, welche entlang einer längsten Kante eines kleinsten gedachten Quaders verläuft, welcher das Objekt gerade noch vollständig umschließt.

Ferner können der proximale Abschnitt des Schafts und und der distale Abschnitt des Schafts als separate Bauteile ausgeführt sein, die miteinander verliersicher aneinandergekoppelt werden können. Unter "verliersicher" ist eine derartige Ausgestaltung der Schaftabschnitte zu verstehen, dass die Abschnitte nicht versehentlich verloren gehen oder sich voneinander lösen können.

Der distale Abschnitt umfasst beispielsweise einen distalseitigen Endabschnitt, der sich zum Beispiel über höchstens 20%, höchstens 10% oder sogar höchstens 5% der Gesamtlänge des Schafts erstreckt. Der proximale Abschnitt kann gemeinsam mit dem distalen Abschnitt den Schaft ausbilden und/oder sich unmittelbar an den distalen Abschnitt anschließen. Insofern kann der proximale Abschnitt einen Großteil des Schafts ausbilden und insbesondere dahingehend proximal sein, dass er sich proximal bzgl. des distalen Abschnitts befindet.

Die hyperspektrale Bilderfassungsbaugruppe kann zur hyperspektralen Bildgebung eingerichtet sein. Unter "hyperspektraler Bilderfassungsbaugruppe" soll insbesondere eine, vorzugsweise funktionsfähige, Einheit oder ein Teilsystem einer Endoskopvorrichtung, insbesondere eine Unterbaugruppe und/oder eine Konstruktions- und/oder eine Funktionskomponente einer Endoskopvorrichtung, verstanden werden, die aus mehreren Einzelkomponenten besteht. Die hyperspektrale Bilderfassungsbaugruppe kann eine Endoskopvorrichtung und bevorzugt einen distalen Schaftabschnitt, zumindest teilweise, ausbilden. Eine "hyperspektrale Bilderfassungsbaugruppe" ist insbesondere so zu verstehen, dass diese konstruktive Merkmale aufweist, um hyperspektrale Bilder erfassen und hyperspektrale Bilddaten erzeugen zu können. Hyperspektrale Bildgebung bzw. hyperspektrale Bilddaten kann sich dabei insbesondere auf solche Bildgebung beziehen, bei der wenigstens 20, wenigstens 50 oder sogar wenigstens 100 Spektralbänder voneinander unabhängig erfassbar sind und/oder erfasst werden.

Bei hyperspektralen Bildern handelt es sich insbesondere um Bilder bzw. Bilddaten, die räumliche und spektrale Information eines abzubildenden Objektbereichs enthalten. Die spektrale Information bezieht sich dabei insbesondere auf Daten über die Intensität von Licht des abzubildenden Objekts über verschiedene Wellenlängen. Die räumliche Information bezieht sich auf die räumlichen Eigenschaften und die Position des/der Objekts/Objekte im abzubildenden Objektbereich.

Als "Bilder" sind hierin insbesondere statische und/oder dynamische visuelle Abbildungen eines abzubildenden Objektbereichs zu verstehen. Dynamische visuelle Abbildungen können insbesondere Bewegtbilder sein.

Die Bilderfassungsbaugruppe ist insbesondere dazu eingerichtet, zumindest zweidimensionale räumliche Bilddaten zu erzeugen. Die Bilderfassungsbaugruppe kann dahingehend räumlich auflösend sein, dass sie in zumindest zwei unterschiedliche Raumrichtungen jeweils eine Auflösung von zumindest 100 Bildpunkten, vorzugsweise von zumindest 200 Bildpunkten, bevorzugt von zumindest 300 Bildpunkten und vorteilhaft von zumindest 400 Bildpunkten liefert. Die Bilddaten sind vorzugsweise zumindest dreidimensional, wobei zumindest zwei Dimensionen räumliche Dimensionen sind und/oder wobei zumindest eine Dimension eine spektrale Dimension ist. Aus den Bilddaten können mehrere räumlich aufgelöste Bilder des Objektbereichs gewinnbar sein, die jeweils unterschiedlichen Spektralbändern zugeordnet sind. Die räumliche und spektrale Information der Bilddaten kann derart beschaffen sein, dass daraus für mehrere räumliche Bildpunkte jeweils ein zugehöriges Spektrum gewinnbar ist.

In einigen Ausführungsformen ist die Bilderfassungsbaugruppe dazu eingerichtet, laufend aktualisierte Bilddaten zu erzeugen. Die Bilderfassungsbaugruppe kann beispielsweise dazu eingerichtet sein, die Bilddaten im Wesentlichen in Echtzeit zu erzeugen, was beispielsweise eine Erzeugung aktualisierter Bilddaten wenigstens als 30 Sekunden, in einigen Fällen wenigstens als 20 Sekunden und in manchen Fällen sogar wenigstens alle 10 Sekunden oder wenigstens alle 5 Sekunden umfasst.

Gemäß einer Weiterbildung kann der distale Schaftabschnitt ein distales Endstück umfassen, in dem die hyperspektrale Bilderfassungsbaugruppe angeordnet ist. In anderen Worten kann die hyperspektrale Bilderfassungsbaugruppe in einem unmittelbar an eine Endoskopspitze angrenzenden und/oder zumindest diese umfassenden Schaftabschnitt angeordnet sein. Als Endoskopspitze ist etwa der äußerst distale Abschnitt des distalen Schaftabschnitts zu verstehen. Auf diese Weise ist die Bilderfassungsbaugruppe in einem Schaftabschnitt angeordnet, der während einer therapeutischen und/oder chirurgischen Aktion, einem zu beobachtenden Objektbereich räumlich betrachtet am nächsten ist. Die Anordnung im distalen Endstück gestattet zudem weitgehende gestalterische Freiheit bzgl. der Ausgestaltung des proximalen Abschnitts und des distalen Abschnitts. Insbesondere kann beispielsweise der Schaft zumindest abschnittsweise flexibel ausgestaltet sein, insbesondere in einem Bereich proximal des distalen Endstücks.

In einer Weiterbildung kann die Endoskopvorrichtung eine Kamerabaugruppe umfassen. Hierdurch können zusätzlich zu hyperspektralen Bildern weitere Bilder wie beispielsweise Weißlichtbilder oder Fluoreszenzbilder aufnehmbar sein. Ein Benutzer kann dadurch einen hyperspektral abzubildenden Objektbereich zusätzlich beobachten und/oder einfach identifizieren und/oder beurteilen. Die Kamerabaugruppe kann eine Einheit oder ein Teilsystem einer Endoskopvorrichtung, insbesondere eine Unterbaugruppe und/oder eine Konstruktions- und/oder eine Funktionskomponente darstellen. In einer Ausführungsform kann die Kamerabaugruppe zusätzlich zur hyperspektralen Bilderfassungsbaugruppe ausgebildet sein. Die Kamerabaugruppe kann mit all ihren Funktionskomponenten in dem distalen Schaftabschnitt und insbesondere in dem distalen Endstück des Schafts angeordnet sein. Beispielsweise kann die Kamerabaugruppe ein Kameracube sein. Ferner kann die Kamerabaugruppe wenigstens eine, insbesondere erste, Bilderfassungssensorik mit wenigstens einem Bildsensor umfassen, der dazu eingerichtet ist, Bilder zu erfassen und Bilddaten zu erzeugen. Der wenigstens eine Bildsensor kann insbesondere als Farbbildsensor bzw. Sensorik zur Weißlichtbilderfassung ausgebildet sein.

Die Endoskopvorrichtung kann ferner eine Beleuchtungsvorrichtung umfassen und/oder an eine solche anschließbar sein, die zumindest ein Leuchtmittel umfasst, das dazu eingerichtet ist, in zumindest einem Betriebszustand den Objektbereich zu beleuchten und/oder auszuleuchten. Das Leuchtmittel kann eine Weißlichtquelle, eine, insbesondere durchstimmbare, monochrome Leuchtquelle, ein Laser, ein Weißlichtlaser, zumindest eine Leuchtdiode und/oder ein Leuchtdiodenarray, zumindest, eine Laserdiode und/oder ein Laserdiodenarray oder dergleichen umfassen. Die Beleuchtungsvorrichtung kann mit der Bilderfassungssensorik integral ausgebildet sein. Insbesondere kann die Beleuchtungsvorrichtung einzelne oder sämtliche Komponenten der Optik der Endoskopvorrichtung nutzen und/oder über eine hiervon separate Beleuchtungsoptik verfügen. Ein Beleuchtungslichtstrahl kann zumindest abschnittsweise koaxial mit einem Messlichtstrahl geführt und/oder führbar sein.

Die Endoskopvorrichtung kann wenigstens eine Eingangsoptik umfassen, durch die Abbildungslicht einkoppelbar ist. Die Eingangsoptik kann eine Blickrichtung definieren. Diese kann etwa durch eine optische Achse der Eingangsoptik definiert und insbesondere parallel und/oder koaxial mit dieser sein. Die Blickrichtung kann parallel zu einer Längsachse des Schafts und/oder des distalen Abschnitts und/oder des distalen Endstücks oder relativ zu dieser schräg bzw. winklig orientiert sein. Beispielsweise kann sie mit der betreffenden Längsachse einen Winkel von etwa 15 Grad, 30 Grad, 45 Grad oder 60 Grad einschließen. Die Eingangsoptik kann beispielsweise ein Objektiv umfassen. In einigen Ausführungsformen kann die Eingangsoptik distalseitig an dem distalen Endstück angebracht sein. Die Eingangsoptik kann in einigen Ausführungsformen einen Durchmesser aufweisen, der wenigstens dem Radius des distalen Endabschnitts entspricht und insbesondere größer als dieser ist. Hierdurch kann ein hoher Grad an Effizienz bei der Lichteinkopplung erzielbar sein.

In einigen Ausführungsformen kann die Endoskopvorrichtung ferner dazu eingerichtet sein, die Blickrichtung bzw. den Blickwinkel, insbesondere während eines Eingriffs an einem Patienten, zu variieren. Dazu kann die Endoskopvorrichtung beispielsweise Schwenkoptiken und/oder einen schwenkbaren Schaftabschnitt aufweisen, die variable Blickrichtungen ermöglichen, und/oder eine digitale Schwenkoptik, die durch entsprechende Bildverarbeitung unterschiedliche Blickrichtungen ermöglicht. Die Betrachtung des Objektbereichs aus verschiedenen Blickwinkeln bietet den Vorteil, eine umfassende und dreidimensionale Perspektive, also einen Rundumblick, zu erhalten. Dadurch können detaillierte Informationen über beispielsweise eine Form, Struktur bzw. Eigenschaften des zu betrachtenden Objekts sowie dessen Umgebung gewonnen werden.

Eine Weiterbildung betrifft zudem eine Endoskopvorrichtung, die wenigstens eine Eingangsoptik und wenigstens einen Strahlteiler, umfasst. Hierdurch kann eine bauliche Einfachheit erzielt werden. Zudem kann hierdurch Abbildungslicht wirksam und mit hoher Effizienz eingekoppelt und dennoch unterschiedlichen Abbildungspfaden bzw. Bildsensoren zuführbar sein. Die wenigstens eine Eingangsoptik kann dazu eingerichtet sein, von dem Objektbereich kommendes Objektlicht bzw. Abbildungslicht zu der Bilderfassungssensorik zu führen, beispielsweise es zu fokussieren und/oder zu projizieren. Das Objektlicht kann insbesondere von einer Beleuchtung des Objektbereichs herrühren. Die wenigstens eine Eingangsoptik kann sowohl für die hyperspektrale Bilderfassungsbaugruppe als auch für die Kamerabaugruppe als eine gemeinsame Optik verwendet werden. In einigen Ausführungsformen können die hyperspektrale Bilderfassungsbaugruppe und die Kamerabaugruppe auch separate Eingangsoptiken bzw. Objektive aufweisen.

Der Strahlteiler kann dabei hinter der Eingangsoptik angeordnet und dazu eingerichtet sein einen ersten Anteil einfallenden Lichts der hyperspektralen Bilderfassungsbaugruppe zuzuführen und einen zweiten Anteil einfallenden Lichts der Kamerabaugruppe zuzuführen. Somit kann ein einzelner Eingangsstrahlengang verwendet werden, auch wenn unterschiedliche Bildsensoren und/oder Bildgebungsmethoden verwendet werden. In Ausführungsformen, in denen die Endoskopvorrichtung mehr als eine Eingangsoptik aufweist, kann ein Strahlteiler obsolet sein. Der Strahlteiler kann insbesondere dazu eingerichtet sein, wellenlängenselektiv zu sein. Insbesondere kann es sich bei dem Strahlteiler um einen dichroitischen Spiegel handeln, in einigen Ausführungsformen um einen wellenlängenselektiven dichroitischen Spiegel. Darüber hinaus kann der Strahlteiler dazu eingerichtet sein, der Kamerabaugruppe sichtbares Licht, insbesondere zumindest in einem Wellenlängenbereich von 450 nm bis 650 nm und vorzugsweise zumindest in einem Wellenlängenbereich von 420 nm bis 700 nm, zuzuführen und der hyperspektralen Bilderfassungsbaugruppe Infrarotlicht und/oder UV-Licht zuzuführen, insbesondere zumindest Licht mit einer Wellenlänge von über 1500 nm und unter 450 nm, bevorzugtLicht mit einer Wellenlänge von über 1000 nm und unter 420 nm und besonders bevorzugt Licht mit einer Wellenlänge von über 800 nm und unter 400 nm. Zweckmäßig können unterschiedliche Strahlteiler mit verschiedenen optischen Eigenschaften eingesetzt werden. Insbesondere können sich unterschiedliche Strahlteiler hinsichtlich ihres Brechungsindexes, der Reflexion, der Transmission, der Absorption, der Dispersion sowie der Streuung unterscheiden. In einigen Ausführungsformen kann der Strahlteiler wechselbar sein. Hierdurch kann beispielsweise eine Anpassung an einen jeweils relevanten Spektralbereich dadurch ermöglicht werden, dass ein Strahlteiler ausgewählt wird, der im relevanten Spektralbereich Licht geeignet reflektiert bzw. transmittiert.

Gemäß einer weiteren Ausführung kann die hyperspektrale Bilderfassungsbaugruppe eine Blende umfassen, die dazu eingerichtet ist, das Abbildungslicht gemäß einem Blendenmuster auszublenden und durchzulassen. Zusätzlich kann die Bilderfassungsbaugruppe eine Aufspaltungsoptik umfassen, die dazu eingerichtet ist, durch die Blende durchgelassenes Abbildungslicht spektral aufzuspalten. In einigen Ausführungsformen kann die Aufspaltungsoptik beispielsweise als ein Prisma oder als optisches Gitter ausgebildet sein. Die Art sowie die Anzahl der Aufspaltungsoptiken kann insbesondere in Abhängigkeit unterschiedlicher Blendenmuster variieren.

Ferner kann die hyperspektrale Bilderfassungsbaugruppe wenigstens eine, insbesondere zweite, Bilderfassungssensorik umfassen, die einen lichtempfindlichen Bereich definiert und die bezüglich der Aufspaltungsoptik derart angeordnet ist, dass spektral aufgespaltetes Licht auf den lichtempfindlichen Bereich fällt. Die zweite Bilderfassungssensorik der hyperspektralen Bilderfassungsbaugruppe kann dabei zusätzlich und separat zu der ersten Bilderfassungssensorik der Kamerabaugruppe ausgebildet sein. Die Eingangsoptik, die Blende, die Aufspaltungsoptik und die Bilderfassungssensorik können in dem distalen Schaftabschnitt angeordnet sein. Es versteht sich, dass in einigen Ausführungsformen lediglich die hier als "zweite" Bilderfassungssensorik bezeichnete Bilderfassungssensorik vorhanden sein kann, beispielsweise dann, wenn keine Kamerabaugruppe vorhanden ist.

In einer alternativen Ausführungsform der Endoskopvorrichtung können die Kamerabaugruppe und die hyperspektrale Bilderfassungsbaugruppe eine gemeinsame Bilderfassungssensorik aufweisen. Eine derartige Konfiguration ermöglicht eine sehr kompakte und effiziente Bauweise, was sich nicht nur positiv auf die Herstellungskosten und die Effizienz der Bilderfassung auswirkt, sondern sich auch neue Einsatzbereiche ergeben können.

Um das Abbildungslicht gezielt umzulenken oder auszurichten, können weitere Ausführungsformen zusätzlich zu der Eingangsoptik und der Aufspaltungsoptik weitere optische Elemente, wie beispielsweise Umlenkspiegel oder Kollimatorlinsen, vorgesehen werden.

Die Endoskopvorrichtung und insbesondere die erste und/oder zweite Bilderfassungssensorik ist zur wenigstens bildabschnittsweisen multispektralen Bildgebung, insbesondere Weißlichtbildgebung und/oder hyperspektralen Bildgebung eingerichtet, im Speziellen dazu, wenigstens bildabschnittsweise multispektrale und/oder hyperspektrale Bilddaten zu erfassen und/oder zu erzeugen. Die Weißlichtbildgebung und die hyperspektrale Bildgebung können gleichzeitig und/oder abwechselnd durchgeführt werden. Zudem können diese zeitweise gleichzeitig und/oder zeitweise nacheinander durchgeführt werden. Multispektrale Bildgebung bzw. multispektrale Bilddaten kann sich dabei insbesondere auf solche Bildgebung beziehen, bei der wenigstens drei Spektralbänder voneinander unabhängig erfassbar sind und/oder erfasst werden. "Bildabschnittsweise" kann bedeuten, dass für zumindest einen Teilbereich eines erfassten Bildes zusätzlich zu räumlicher Information spektrale Information erfassbar ist, beispielsweise für einen oder mehrere Bildpunkte und/oder Bildstreifen.

Die Bilderfassungssensorik kann zumindest einen Bildsensor aufweisen. Der zumindest eine Bildsensor kann den lichtempfindlichen Bereich definieren. Der Bildsensor kann ein Siliziumsensor sein, beispielsweise ein CCD-Sensor oder ein CMOS-Sensor. Der Bildsensor kann ein zweidimensionales Pixelmuster aufweisen. Allgemein ausgedrückt kann die Bilderfassungssensorik Bildabschnitte definieren, die in dem lichtempfindlichen Bereich angeordnet sind und diesen insbesondere gemäß einem Pixelmuster abdecken. Der lichtempfindliche Bereich kann ein Bilderfassungssensorbereich sein, der von einem oder mehreren Bildsensoren definiert ist. Der lichtempfindliche Bereich ist in einigen Ausführungsformen rechteckig, wobei eine erste Seite des lichtempfindlichen Bereichs zumindest im Wesentlichen parallel zu der Raumachse orientiert sein kann, entlang derer die spektrale Aufspaltung erfolgt, und wobei eine zweite Seite zumindest im Wesentlichen senkrecht zu der ersten Seite orientiert sein kann. Abbildstreifen, die sich parallel zu der zweiten Seite erstrecken, können somit monochromatischen bzw. schmalbandig aufgenommenen räumlichen Bildstreifen entsprechen. Insbesondere sind die Abbildstreifen oder auch Abbildpunkte durch die Blende bzw. das Blendenmuster definiert. Abbildstreifen, die sich parallel zu der ersten Seite erstrecken, können Spektren eines bestimmten Abbildpunkts entsprechen. Die erste Seite kann länger sein als die zweite Seite. Der Begriff "Bildsensor" kann sich auf ein vollständiges elektronisches Bauelement beziehen. Ein Bildsensor im Sinne dieser Offenbarung kann entsprechend neben einem Halbleiterchip zugehörige Kontakte, Leiterbahnen, Rahmen und/oder Strukturelemente umfassen.

Insbesondere die Bilderfassungssensorik aber auch jede andere Wärmequelle innerhalb der Endoskopvorrichtung, wie beispielsweise Leuchtmittel, können mit einer Kühlvorrichtung ausgestattet sein. Durch Regelung und insbesondere die Senkung der Betriebstemperaturen der Bilderfassungssensorik kann Bildrauschen reduziert und dadurch die Bildqualität verbessert werden. Ferner werden empfindliche Komponenten thermisch weniger beansprucht, sodass die Lebensdauer der Endoskopvorrichtung verlängert und Instandhaltungskosten gesenkt werden können. Eine für diesen Zweck geeignete Kühlvorrichtung ist beispielsweise in DE 10 2019 129 815 A1 beschrieben.

Die Aufspaltungsoptik kann ein dispersives Element umfassen, das einfallendes Licht je nach Wellenlänge auf unterschiedliche Positionen und somit unterschiedliche Pixel des verwendeten Bildsensors der Bilderfassungssensorik lenkt. Aus der Position eines Pixels kann dann auf die Wellenlänge des Lichts geschlossen werden. In einigen Ausführungsformen kann der betreffende Bildsensor derart angeordnet sein, dass eine Richtung der dispersiven Aufspaltung parallel zu einer der beiden Bildachsen des Bildsensors verläuft. Eine zusätzliche Abbildungslinse, die zwischen dem Bildsensor und der Aufspaltungsoptik angeordnet sein kann, kann das Licht gezielt auf den Bildsensor lenken.

In einer weiteren Ausführungsform können ein Strahlengang, der von dem Strahlteiler zu dem Bildsensor der Kamerabaugruppe verläuft und ein Strahlengang, der von dem Strahlteiler zu der Bilderfassungssensorik der hyperspektralen Bilderfassungsbaugruppe verläuft, zumindest abschnittsweise zumindest im Wesentlichen parallel zueinander verlaufen. Dies kann bedeuten, dass Lichtstrahlen zueinander versetzt in einer geraden Linie verlaufen und sich nicht kreuzen oder konvergieren. Anders ausgedrückt können die genannten Strahlengänge abschnittsweise nebeneinander verlaufen.

In einigen Ausführungsformen einer Endoskopvorrichtung können die Kamerabaugruppe und die hyperspektrale Bilderfassungsbaugruppe bezüglich einer Längsachse des distalen Abschnitts des Schafts zumindest abschnittsweise nebeneinander angeordnet sein. Eine Aufspaltung in nebeneinander verlaufende Strahlengänge ist dann baulich einfach umzusetzen. Die Anordnung nebeneinander gestattet es, zwei Eingangsoptiken nebeneinander zu verwenden. Strahlengänge können somit unabhängig voneinander aufgebaut sein. Ebenso ist es denkbar, dass ein Strahlteiler verwendet wird, wenn die Kamerabaugruppe und die hyperspektrale Bilderfassungsbaugruppe nebeneinander angeordnet sind. Es kann dann zur Lichteinkopplung eine einzelne Eingangsoptik verwendet werden, die unterschiedlichen Strahlengänge aber getrennt voneinander aufgebaut sein. Generell kann eine kurz bauende Anordnung von Baugruppen bereitgestellt werden.

In alternativen Ausführungsformen können die Kamerabaugruppe sowie die hyperspektrale Bilderfassungsbaugruppe bezüglich der Längsachse des distalen Abschnitts des Schafts zumindest abschnittsweise hintereinander angeordnet sein. Hierdurch kann für jeden der verwendeten Bildsensoren der Schaftquerschnitt effektiv ausgenutzt werden. Verfügbarer Bauraum entlang der Längsachse kann hierdurch ausgenutzt werden.

Die Ausführungsformen, bei denen die Kamerabaugruppe und die Bilderfassungsbaugruppe bezüglich einer Längsachse des distalen Abschnitts des Schafts nebeneinander angeordnet sind, haben zudem den Vorteil, dass der Lichtweg des Abbildungslichts hin zu den Bildsensoren sehr kurz ist. Lichtverluste, insbesondere Verluste im IR-Bereich, können bei einer derartigen Anordnung der Baugruppen somit minimiert werden.

Bei der Ausführungsform, in der die Baugruppen hintereinander angeordnet sind, kann durch die platzsparende Anordnung der Schaft der Endoskopvorrichtung optimiert bzw. reduziert werden. So kann der Durchmesser des Schafts der Endoskopvorrichtung vorzugsweise höchstens 15 mm, bevorzugt höchstens 10 mm und besonders bevorzugt höchstens 5 mm betragen.

In einer Weiterbildung der Endoskopvorrichtung kann die Bilderfassungssensorik relativ zu dem distalen Abschnitt des Schafts ortsfest sein. Ebenso kann die Eingangsoptik und/oder die Aufspaltungsoptik relativ zu dem distalen Abschnitt des Schafts ortsfest sein. Ferner kann die Bilderfassungssensorik relativ zu der Eingangsoptik unbeweglich sein. In anderen Worten weist eine derartige Ausführungsform einer Endoskopvorrichtung keine Verlagerungseinheit auf, die dazu eingerichtet ist, die Kamerabaugruppe und/oder die hyperspektrale Bilderfassungsbaugruppe relativ zum abzubildenden Objektbereich zu verlagern bzw. zu bewegen und/oder zu verschieben. Durch den Wegfall einer solchen Verlagerungseinheit, die bei herkömmlichen Bildgebungsvorrichtungen, die nach dem Pushbroom- oder dem Whiskbroom-Prinzip funktionieren, unabdingbar ist, kann die Bildgebungsvorrichtung wesentlich kompakter ausgestaltet werden und der Zugang zum Objektbereich für einen behandelnden Arzt erleichtert werden. Zudem kann eine Endoskopvorrichtung bereitgestellt werden, die wenig anfällig für Erschütterungen und Bewegungen ist, sodass entsprechende Artefakte vermieden werden können, die im Fall von Pushbroom- sowie Whiskbroom-Anordnungen aufgrund des vergleichsweisen langsamen und aufwändigen mechanischen Scannens auftreten können.

Gemäß einer weiteren Ausführungsform kann die Blende der Bilderfassungsbaugruppe als eine Lochblende oder als Schlitzblende ausgebildet sein, die mehrere vorzugsweise parallele Reihen von Blendenlöchern und/oder Schlitzen umfasst. Die folgenden Ausführungen zu Blendenlöchern bzw. Lochreihen sind analog auch auf Schlitze bzw. auf Blenden mit Schlitzen anwendbar.

Die Blendenlöcher sind dazu eingerichtet, Bildabschnitte bzw. Teilabschnitte des zu beobachtenden Objektbereichs zu definieren und den Lichtfluss des Abbildungslichts selektiv zu steuern. Die Bildabschnitte können beispielsweise wenigstens 1%, wenigstens 2%, wenigstens 5% oder wenigstens 20% einer Gesamtbildfläche ausmachen. Die Reihen von Blendenlöchern können bezüglich einer Querrichtung der Aufspaltungsoptik und/oder des lichtempfindlichen Bereichs schräg verlaufen. Ferner können die Reihen von Blendenlöchern um einen Lochreihenabstand voneinander beabstandet sein. Die hyperspektrale Bilderfassungsbaugruppe kann ferner einen auflösbaren Wellenlängenbereich definieren. Die Aufspaltungsoptik kann ferner dazu eingerichtet sein, den auflösbaren Wellenlängenbereich entlang einer räumlichen Achse über einen Aufspaltungsabstand aufzuspalten, der höchstens so groß ist wie der Lochreihenabstand. Eine derartige Konfiguration verhindert, dass sich mehrere Spektren unterschiedlicher Bildpunkte überlagern.

Es versteht sich, dass prinzipiell eine Vielzahl unterschiedlicher Blendenmuster mit unterschiedlichen Eigenschaften eingesetzt werden können. Das eingesetzte Blendenmuster kann daher je nach Einsatzbereich und Anforderungen variieren und entsprechend ausgetauscht werden.

In einer Weiterbildung kann die Blende relativ zu der Bilderfassungssensorik bewegbar gelagert sein. Mit einer derartigen Konfiguration gelingt es, die räumliche Auflösung und damit die Qualität des Bilds zu erhöhen. Insbesondere in Kombination mit einer Blende, die mehrere Lochreihen und/oder Schlitze aufweist, können hierdurch einerseits mehrere Bildzeilen aufgenommen werden, zugleich aber für jede Bildzeile eine hinreichende spektrale Auflösung erreicht werden. Einzelspektren einzelner Punkte treten dann nach Maßgabe der Anordnung der Lochreihen oder Schlitze nebeneinander auf. Ein vollständiges Bild kann hierbei aufgenommen werden, indem die Blende zwischen Einzelbildaufnahmen relativ zur Bilderfassungssensorik bewegt wird. Wiederum können dann mehrere Bildzeilen gleichzeitig hyperspektral abgebildet werden. In einigen Ausführungsformen kann auch eine Blende mit einer einzigen Lochreihe und/oder einem einzigen Schlitz verwendet werden. In diesem Fall kann die Blende dann über einen Abstand verlagerbar sein, der hinreichend groß ist, um den durch die einzige Lochreihe und/oder den einzigen Schlitz hindurch auf die Bilderfassungssensorik geführten Lichtstreifen über den gesamten lichtempfindlichen Bereich der Bilderfassungssensorik oder zumindest über einen Großteil desselben bewegen zu können. Aus hierbei aufgenommenen Einzelbildern kann dann ein hyperspektraler Datensatz gewonnen werden. Die Bilderfassungsbaugruppe kann eine Blendenverlagerungseinheit mit einem Aktor und einem Blendenträger umfassen. In einer Weiterbildung können die Blende und der Blendenträger einstückig ausgebildet sein. Der Blendenträger kann mit dem Aktor verbunden sein, sodass die Blende mittels des Aktors relativ zu der Bilderfassungssensorik bewegbar ist.

Ferner kann der Blendenträger ein Festkörpergelenk umfassen. Dieses kann zuverlässig, belastbar und/oder kostengünstig sein. Das Festkörpergelenk definiert vorzugsweise eine einzelne Schwenkachse, welche die Bewegung der Blende definiert.

Gemäß einer Weiterbildung kann der Aktor dazu eingerichtet sein, eine Linearbewegung zu erzeugen. Diese Linearbewegung kann mittels des Festkörpergelenks in eine Schwenkbewegung der Blende transformiert werden. Dabei ist die Blendenverlagerungseinheit bzw. die Blende vorzugsweise derart konfiguriert, dass die Richtung der Schwenkbewegung ungleich den Erstreckungsrichtungen sich entlang der Lochreihen bzw. Spalten erstreckenden Achsen erfolgt. Besonders bevorzugt erfolgt die Schwenkbewegung senkrecht oder schräg zu den sich entlang der Lochreihen bzw. Spalten erstreckenden Achsen. Der Aktor kann insbesondere als Piezoaktor ausgeführt sein.

Vorhandener Bauraum kann insbesondere dann effizient ausgenutzt werden, wenn der Aktor bezüglich einer Längsachse des distalen Abschnitts von der Eingangsoptik aus betrachtet hinter der Bilderfassungssensorik angeordnet ist, sodass der Blendenträger die Bilderfassungssensorik überspannt. Hierdurch kann der Aktor bzgl. der Bilderfassungssensorik proximal angeordnet sein und somit den Lichtweg zu Bilderfassungssensorik unbeeinträchtigt lassen. Der Blendenträger kann somit als Hebel wirken, der die Blende trägt. Der Blendenträger kann um die von dem Festkörpergelenk definierte Schwenkachse schwenkbar sein.

In einer Ausführungsform kann die Verlagerung des Blendenträgers bzw. der Blende erfolgen, ohne, dass sich dabei auch die Bilderfassungssensorik verschiebt. Somit verschieben sich während des Verlagerungsvorgangs die Blendenlöcher und die Bilderfassungssensorik relativ zueinander. Diese Relativbewegung hat je nach Konfiguration des Bildsensors zur Folge, dass mit zunehmender Verlagerung immer weniger Licht des abzubildenden Objektbereichs auf den Bildsensor der Bilderfassungssensorik fällt. Damit trotzdem ausreichend Spektralinformation von der Bilderfassungssensorik erfasst wird, kann/können die Blende bzw. die Blendenlöcher entlang einer Bewegungsstrecke über eine maximale Distanz bewegbar sein, bei welcher der aufgelöste Wellenlängenbereich der Bildpunkte um höchstens 1000 %, vorzugsweise höchstens 800 %, bevorzugt höchstens 600 % und besonders bevorzugt höchstens 400 % einer Breite und/oder um höchstens 100 %, vorzugsweise höchstens 80 %, bevorzugt höchstens 60 % und besonders bevorzugt höchstens 20 % der Länge des einen Bildpunkt definierenden Pixelmusters verschoben wird. Auf diese Weise lässt sich die räumliche Auflösung schnell und mit geringem technischem Aufwand verbessern, sodass ausreichend und aussagekräftige Informationen über den abzubildenden Objektbereich erfasst werden können. Die Qualität der Auflösung hängt dabei unter anderem maßgeblich von der Anzahl der Blendenlöcher, beziehungsweise der Anzahl der Pixel des Detektorarrays, sowie dem Betrag der Bewegungsstrecke der Blende ab.

Gemäß einer weiteren Ausführungsform kann die Blende um eine Rotationsachse drehbar gelagert sein, die zumindest im Wesentlichen parallel oder koaxial zu einer optischen Achse der Blende angeordnet ist. Die Blende kann dabei insbesondere als eine Schlitzblende ausgebildet sein, deren Beobachtungsschlitz dazu eingerichtet ist, zumindest einen und bevorzugt eine Vielzahl unterschiedlicher Bildstreifen eines durch die Eingangsoptik erzeugten Zwischenbilds eines abzubildenden Objektbereichs auszuwählen. Ferner kann die Bilderfassungsbaugruppe ein Strahlendreher-Element, insbesondere in Form eines Dove-Prismas umfassen, das gemeinsam und/oder getrennt mit/von der Blende drehbar gelagert ist. Das Strahlendreher-Element kann dazu eingerichtet sein, die Strahlen des durch die Aufspaltungsoptik spektral aufgespaltenen Lichts, so zu drehen, dass diese, unabhängig von der Position des Blendenschlitzes, von der Bilderfassungssensorik detektiert werden können, ohne dabei das Lichtspektrum zu beeinflussen. In anderen Worten dient das Strahlendreher-Element dazu, die Strahlen stets in derselben Ausrichtung auf die Aufspaltungsoptik zu auszurichten. Vorzugsweise verläuft die Rotationsachse der Blende koaxial mit der Längsachse des Strahlendreher-Elements.

Des Weiteren kann die Bilderfassungsbaugruppe wenigstens einen Drehantrieb umfassen, der dazu eingerichtet ist, die Blende und/oder das Strahlendreher-Element um die Rotationsachse zu drehen. Durch eine solche Anordnung kann eine zur Auswahl unterschiedlicher Bildstreifen erforderliche Bewegung einfach, zuverlässig und reproduzierbar erzeugt werden. Vorteilhafterweise verläuft die Rotationsachse durch den Schwerpunkt des Strahlendreher-Elements. Darüber hinaus können die Blende und/oder das Strahlendreher-Element in zwei entgegengesetzten Richtungen um die wenigstens eine Rotationsachse rotierbar sein. Ferner kann die Rotationsbewegung oszillatorisch, insbesondere gemäß einer Pendelbewegung, oder kontinuierlich erfolgen. Kontinuierliche Bewegungen sind im Vergleich zu beispielsweise oszillierenden Bewegungen wesentlich stabiler, da sie weniger anfällig für Störungen oder Unregelmäßigkeiten sind. Ferner können durch eine kontinuierliche Bewegung Pendelbewegungen und damit verbundene Belastungen aufgrund periodischer Beschleunigung vermieden werden. Insbesondere kann eine gleichmäßige Rotationsgeschwindigkeit beibehalten werden. Je nach Anwendungsbereich kann daher eine kontinuierliche Drehbewegung des optischen Elements bevorzugt werden. Je nach Anwendungsgebiet sowie dessen Anforderungen an eine Endoskopvorrichtung, kann zwischen unterschiedlichen Antriebsarten, Rotationsgeschwindigkeiten und/oder Winkelbeträgen variiert werden.

In einer Weiterbildung kann die hyperspektrale Bilderfassungsbaugruppe eine Steuereinheit umfassen, die dazu eingerichtet ist, das die Blende und/oder das Strahlendreher-Element verlagernde Antriebselement jeglicher Art und die Bilderfassungssensorik anzusteuern. Diese Ansteuerung kann synchronisiert erfolgen. Die Bilderfassungssensorik kann ferner dazu eingerichtet sein, eine Datenerfassung, insbesondere eine kontinuierliche Datenerfassung, durchzuführen, die mit der kontinuierlichen Bewegung der Blende und/oder des Strahlendreher-Elements synchronisiert ist. So können die von der Bilderfassungssensorik erfassten Daten stets einer Position der Blende und damit einer Position auf dem Objektbereich zugeordnet werden. Die Bilderfassungssensorik kann beispielsweise dazu eingerichtet sein, die Bilddaten im Wesentlichen in Echtzeit zu erzeugen. Eine Bilderfassungsrate der Bilderfassungssensorik kann beispielsweise wenigstens 1 fps, 10 fps, 20 fps, 30 fps, 50 fps oder sogar 100 fps betragen.

Gemäß einer weiteren Ausführungsform kann die hyperspektrale Bilderfassungseinheit eine optische Zwischenbildverlagerungseinheit umfassen, die dazu eingerichtet ist, das Zwischenbild relativ zu der Blende und insbesondere relativ zu dem Beobachtungsspalt der Blende optisch zu verlagern, sodass mit der Blende unterschiedliche Zwischenbildabschnitte des Zwischenbilds ausgewählt werden können. Die optische Zwischenbildverlagerungseinheit kann ein bewegliches optisches Element umfassen, das dazu eingerichtet ist, die Position des Zwischenbilds relativ zu dem Beobachtungsspalt zu verlagern. Eine derartige optische Zwischenbildverlagerungseinheit ermöglicht das Abscannen eines erweiterten Objektbereichs, der außerhalb des Objektbereichs liegt, dessen Zwischenbild auf einen positionstreuen Beobachtungsspalt der Blende fällt bzw. der von einer positionstreuen Bilderfassungsbaugruppe erfasst wird. Auf diese Weise kann die Position des Zwischenbildes schnell und mit geringem technischem Aufwand verlagert werden, um ausreichend Informationen über den abzubildenden Objektbereich zu erfassen.

In einer Weiterbildung kann das optische Element zwischen der Eingangsoptik und der Blende angeordnet sein. Ferner kann das optische Element ein Prisma und insbesondere ein wenigstens vierseitiges Prisma umfassen. Jenes Prisma kann spezifische optische Eigenschaften aufweisen. Zweckmäßig können unterschiedliche Prismen mit verschiedenen optischen Eigenschaften eingesetzt werden. Insbesondere können sich unterschiedliche Prismen hinsichtlich ihres Brechungsindexes, der Reflexion, der Transmission, der Absorption, der Dispersion sowie der Streuung unterscheiden.

Darüber hinaus kann die hyperspektrale Bilderfassungssensorik dazu eingerichtet sein, während einer kontinuierlichen Bewegung des optischen Elements kontinuierlich hyperspektrale Bilddaten zu erfassen. Die Datenerfassung der hyperspektralen Bilderfassungssensorik kann mit der kontinuierlichen Bewegung des optischen Elements synchronisiert sein. So können die von der Bilderfassungssensorik erfassten Daten stets einer Position des optischen Elements und damit einer Position auf dem Objektbereich zugeordnet werden. Die Bilderfassungssensorik kann beispielsweise dazu eingerichtet sein, die Bilddaten im Wesentlichen in Echtzeit zu erzeugen. Eine Bilderfassungsrate der Bilderfassungssensorik kann beispielsweise wenigstens 1 fps, 10 fps, 20 fps, 30 fps, 50 fps oder sogar 100 fps betragen.

In einer weiteren Ausführungsform kann die Zwischenbildverlagerungseinheit einen Antrieb für das optische Element umfassen, der dazu eingerichtet ist, das optische Element in eine vorgegebene Drehrichtung um insbesondere wenigstens 90 Grad und vorzugsweise zumindest 360 Grad zu drehen. Die Drehrichtung und/oder die Drehgeschwindigkeit und/oder der Verdrehwinkel können variierbar sein. Besonders bevorzugt kann die Rotationsbewegung kontinuierlich sein. Durch eine kontinuierliche Bewegung können Pendelbewegungen und damit verbundene Belastungen aufgrund periodischer Beschleunigung vermieden werden. Insbesondere kann eine gleichmäßige Rotationsgeschwindigkeit beibehalten werden. Ferner sind kontinuierliche Bewegungen im Vergleich zu beispielsweise oszillierenden Bewegungen wesentlich stabiler, da sie weniger anfällig für Störungen oder Unregelmäßigkeiten sind. Je nach Anwendungsbereich kann daher eine kontinuierliche Drehbewegung des optischen Elements bevorzugt werden.

Alternativ kann der Antrieb beispielsweise als Pendelantrieb ausgestaltet und dazu eingerichtet sein, das optische Element oszillatorisch und insbesondere gemäß einer Pendelbewegung zu bewegen. Je nach Anwendungsgebiet sowie dessen Anforderungen an eine Endoskopvorrichtung, kann zwischen den unterschiedlichen Antriebsarten variiert werden. Der Antrieb kann insbesondere über eine mit dem optischen Element drehmomentübertragend gekoppelte Motoreinheit initiiert und gesteuert werden.

In einigen Ausführungsformen kann die Endoskopvorrichtung eine Haltevorrichtung umfassen, die dazu eingerichtet ist, die Kamerabaugruppe und/oder die hyperspektrale Bilderfassungsbaugruppe zu halten. Die Haltevorrichtung kann derart konfiguriert sein, dass sie in dem distalen Endstück des Schafts angeordnet werden kann. Insbesondere kann ein Schienensystem und/oder eine mechanische und/oder magnetische Arretiervorrichtung vorgesehen sein, mit der die Haltevorrichtung reversibel mit dem Schaft gekoppelt werden kann. Alternativ oder zusätzlich kann das distale Endstück an der Außenumfangsfläche des Schafts eine Zugangsvorrichtung aufweisen, durch welche die Baugruppen bei Bedarf für einen Benutzer zugänglich gemacht werden kann. Sowohl die Haltevorrichtung als auch die Zugangsvorrichtung können insbesondere die Herstellung, Wartungsarbeiten und/oder Reparaturen erleichtern.

Ein weiterer Aspekt betrifft zudem ein medizinisches System, das eine erfindungsgemäße Endoskopvorrichtung umfasst. Des Weiteren umfasst das medizinische System eine Darstellungserzeugungseinheit, die dazu eingerichtet ist, eine Darstellung für einen Benutzer zu erzeugen, die auf einem Zusammenfügen von Information unterschiedlicher Bildpunkte und/oder Bildstreifen zu einem Bildteilbereich beruht. Ferner kann die Darstellung eine Überlagerung des Bildteilbereichs und eines Bilds, insbesondere eines Weißlichtbilds, des abzubildenden Objektbereichs umfassen.

Das medizinische System kann außerdem eine Analyseeinheit umfassen, die dazu eingerichtet ist, nach Maßgabe von Bilddaten der Bilderfassungssensorik eine Analyse zu erstellen, die auf spektraler Information beruht. Die Analyse kann die Ermittlung zumindest eines physiologischen Parameters, insbesondere eines Perfusionsparameters, umfassen. Die Analyse beruht insbesondere auf punktweise oder bildabschnittsweise durchgeführten Berechnungen, denen jeweils spektrale Information zugrunde liegt, die sich auf den entsprechenden Bildpunkt oder Bildabschnitt bezieht. Beispielsweise kann anhand der Analyse ein Perfusionsgrad ortsaufgelöst ermittelbar sein. Weiterhin kann die Darstellungserzeugungseinheit dazu eingerichtet sein, eine Darstellung für einen Benutzer zu erzeugen, die auf der Analyse beruht. Vorzugsweise nutzt die Analyseeinheit eine Spektralanalyse zur Bestimmung von physiologischen Eigenschaften.

Weiterhin kann die Darstellungserzeugungseinheit dazu eingerichtet sein, eine Darstellung für einen Benutzer zu erzeugen, die auf der Analyse beruht.

Das medizinische System kann ferner eine Ausgabeeinheit umfassen, die dazu eingerichtet ist, die von der Darstellungserzeugungseinheit erzeugten Darstellungen an einen Benutzer auszugeben. Die Ausgabeeinheit kann einen Computer und/oder Prozessor und/oder Speicher und/oder Arbeitsspeicher und/oder Anschlüsse und/oder eine Datenschnittschnittstelle zum Empfangen, Verarbeiten und Ausgeben von unverarbeiteten, vorverarbeiteten und/oder verarbeiteten Darstellungsdaten umfassen.

Das medizinische System kann außerdem eine Anzeigeeinheit umfassen, die dazu eingerichtet ist, ein Bild, insbesondere ein Bewegtbild, und insbesondere die von der Bilderfassungseinrichtung aufgenommenen Bilddaten für einen Benutzer anzuzeigen. Die Anzeigeeinheit kann mit der Ausgabeeinheit gekoppelt und insbesondere drahtlos gekoppelt sein. Das angezeigte Bild kann auf den Bilddaten der Bilderfassungssensorik beruhen. Die Anzeigeeinheit kann einen Bildschirm und/oder Steuerungselektronik umfassen. Die Anzeigeeinheit kann einen Computer und/oder Prozessor und/oder Speicher und/oder Arbeitsspeicher und/oder Anschlüsse und/oder eine Datenschnittschnittstelle zum Empfangen, Verarbeiten und Ausgeben von unverarbeiteten, vorverarbeiteten und/oder verarbeiteten Bilddaten und/oder Darstellungsdaten umfassen. Sowohl die Ausgabeeinheit als auch die Anzeigeeinheit können Bestandteile der Darstellungserzeugungseinheit sein.

Das medizinische System kann eine Steuereinheit umfassen. Die Steuereinheit kann geeignete Steuerelektronik und/oder einen Computer umfassen. In einigen Ausführungsformen umfasst die Steuereinheit zumindest einen Prozessor, computerlesbaren Speicher, ein Betriebssystem und/oder geeignete Ein- und Ausgänge. Die Steuereinheit kann zumindest ein Steuerprogramm beinhalten. Insbesondere können Funktionen derselben von dem Steuerprogramm implementiert bzw. Teil desselben sein. Die Endoskopvorrichtung und insbesondere die Steuereinheit kann zur Implementierung der hierin genannten Funktionseinheiten und/oder zur Durchführung von Verfahrensschritten jeweils zumindest einen Prozessor und/oder einen zugeordneten Speicher mit Programmcode, der die beschriebenen Funktionen und Schritte umsetzt, und/oder einen zugeordneten Arbeitsspeicher und/oder zugeordnete Anschlüsse und/oder Datenschnittstellen und/oder eine elektronische Schaltung umfassen. Ein oder mehrere Prozessoren, Speicher, Arbeitsspeicher, Anschlüsse, Datenschnittstellen und/oder Schaltungen können auch einer oder mehreren Funktionseinheiten zugeordnet sein und/oder einen oder mehrere Verfahrensschritte implementieren.

Die erfindungsgemäßen Vorrichtungen und Systeme sollen hierbei nicht auf die oben beschriebene Anwendung und Ausführungsform beschränkt sein. Insbesondere können diese zu einer Erfüllung einer hierin beschriebenen Funktionsweise eine von einer hierin genannten Anzahl von einzelnen Elementen, Bauteilen und Einheiten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

Im Folgenden wird die vorliegende Erfindung anhand der beigefügten Figuren beispielhaft beschrieben. Die Zeichnungen, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es zeigen:
- Fig. 1: eine schematische Darstellung eines medizinischen Systems mit einer Endoskopvorrichtung;
- Fig. 2: eine perspektivische Seitenansicht eines distalen Endstücks der Endoskopvorrichtung gemäß Figur 1;
- Fig. 3: eine schematische Querschnittsansicht eines distalen Endstücks der Endoskopvorrichtung gemäß Figur 2;
- Fig. 4: eine schematische Draufsicht eines distalen Endstücks der Endoskopvorrichtung gemäß Figur 2;
- Fig. 5: eine perspektivische Detailansicht eines Teilbabschnitts der hyperspektralen Bilderfassungsbaugruppe gemäß Figur 2;
- Fig. 6: eine vereinfachte Darstellung der hyperspektralen Bilderfassungsbaugruppe gemäß Figur 2;
- Fig. 7: eine schematische Darstellung eines lichtempfindlichen Bereichs;
- Fig. 8: eine schematische Darstellung einer weiteren Ausführungsform eines distalen Endstücks einer Endoskopvorrichtung;
- Fig. 9: eine schematische Darstellung einer weiteren Ausführungsform eines distalen Endstücks einer Endoskopvorrichtung;
- Fig. 10: eine Verlagerung von Bildpunkten auf einem Objektbereich mit einer Endoskopvorrichtung gemäß Figur 2;
- Fig. 11: eine Überlagerungsdarstellung, die auf einem Weißlichtbild und auf hyperspektralen Bilddaten beruht;
- Fig. 12: eine Überlagerungsdarstellung, die auf einem Weißlichtbild und auf hyperspektralen Bilddaten beruht;
- Fig. 13: eine schematische Darstellung einer weiteren Ausführungsform eines distalen Endstücks einer Endoskopvorrichtung;
- Fig. 14: eine schematische Darstellung einer Blende gemäß Figur 13;
- Fig. 15: eine schematische Darstellung einer weiteren Ausführungsform einer Blende gemäß Figur 13;
- Fig. 16: eine Verlagerung von Bildstreifen auf einem Objektbereich mit einer Endoskopvorrichtung gemäß Figur 13;
- Fig. 17: eine Überlagerungsdarstellung, die auf einem Weißlichtbild und auf hyperspektralen Bilddaten beruht;
- Fig. 18: eine schematische Darstellung einer weiteren Ausführungsform eines distalen Endstücks einer Endoskopvorrichtung;
- Fig. 19: eine schematische Detailansicht einer optischen Zwischenbildverlagerungseinheit gemäß Figur 18;
- Fig. 20: eine Verlagerung von Bildbereichen auf einem Objektbereich mit einer Endoskopvorrichtung gemäß Figur 18;
- Fig. 21: eine Überlagerungsdarstellung, die auf einem Weißlichtbild und auf hyperspektralen Bilddaten beruht;

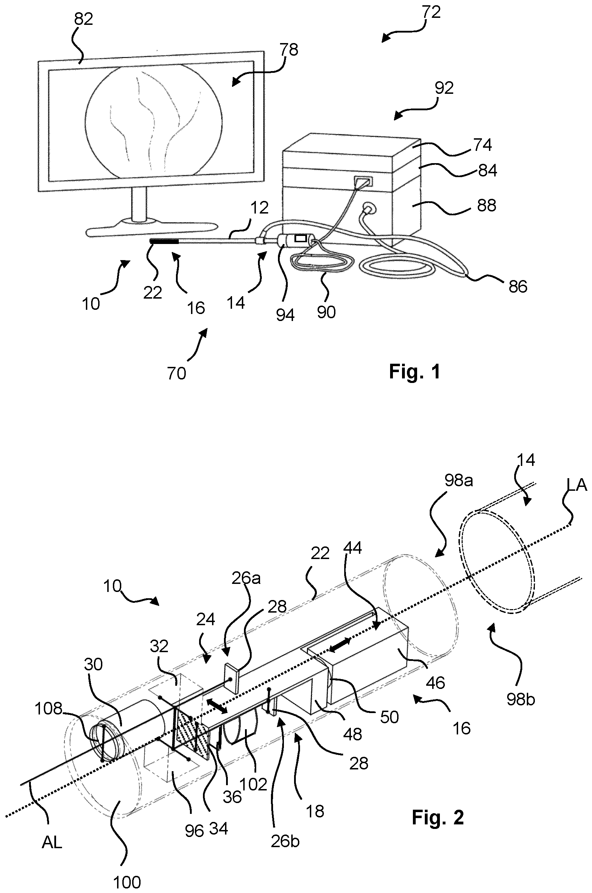

Fig. 1 zeigt eine schematische Darstellung eines medizinischen Systems 72 mit einer Endoskopvorrichtung 10. Die Endoskopvorrichtung 10 ist gemäß der gezeigten Ausführungsform Teil eines Endoskops 70. Die Endoskopvorrichtung 10 umfasst einen Schaft 12 mit einem proximalen Abschnitt 14 sowie einem distalen Abschnitt 16. Der proximale Schaftabschnitt 14 kann mit einem Griff 94 ausgestattet sein, der einem behandelnden Arzt zur sicheren und präzisen Bedienung dient. Der distale Schaftabschnitt umfasst ein distales Endstück 22 des Schafts 12. In diesem distalen Endstück 22 ist eine hyperspektrale Bilderfassungsvorrichtung 18 angeordnet. Diese hyperspektrale Bilderfassungsvorrichtung 18 wird anhand der nachfolgenden Figuren näher beschrieben.

Die Endoskopvorrichtung 10 ist dazu eingerichtet, mithilfe der hyperspektralen Bilderfassungsbaugruppe 18, Bildinformation und insbesondere hyperspektrale Bildinformation eines abzubildenden Objektbereichs 20 während einer therapeutischen und/oder diagnostischen Aktion zu erfassen. Ferner umfasst das System 72 eine Darstellungserzeugungseinheit 74, die dazu eingerichtet ist, eine Darstellung für einen Benutzer zu erzeugen, die auf der erfassten und/oder analysierter Bildinformation beruht. Über eine mit der Darstellungserzeugungseinheit 74 gekoppelten Anzeigeeinheit 82, hier in Form eines Monitors, wird eine Darstellung 78 für einen Benutzer angezeigt. Diese Darstellung 78 zeigt insbesondere eine Überlagerung eines Bildteilbereichs und eines Bildes eines abzubildenden Objektbereichs 20. Die Darstellung 78 beruht ferner auf der Analyse der Bildinformation.

Das System 10 kann wie dargestellt eine Steuervorrichtung 92 mit einer Versorgungseinheit 84 umfassen, an die das Endoskop 16 wahlweise ankoppelbar ist. Die Versorgungseinheit 84 kann dazu eingerichtet sein, das Endoskop 70 zu steuern und/oder Bilddaten und/oder andere Daten von dem Endoskop 70 zu empfangen. Die Versorgungseinheit 84 kann mit der Anzeigeeinheit 82 des Systems 72 verbindbar und/oder verbunden sein, auf der ein Benutzer aufgenommene Bilder darstellen kann. Die Versorgungseinheit 84 kann zudem Beleuchtungslicht für das Endoskop 70 liefern. Das Endoskop 70 kann über ein Lichtleiterkabel 86 an eine Beleuchtungsvorrichtung 88 ankoppelbar und/oder angekoppelt sein. Das Endoskop 70 kann ferner über ein elektrisches Kabel 90 an die Versorgungseinheit 84 ankoppelbar und/oder angekoppelt sein. Das elektrische Kabel 90 kann dazu eingerichtet sein, elektrische Energie und/oder Daten zu übertragen.

In anderen hier nicht gezeigten Ausführungsformen kann eine separate Beleuchtungsvorrichtung und/oder Beleuchtungslichtquelle vorgesehen sein. Zudem kann das Endoskop 70 alternativ oder zusätzlich integrierte Leuchtelemente zur Bereitstellung von Beleuchtungslicht umfassen.

Figur 2 zeigt eine perspektivische Seitenansicht des Aufbaus des distalen Endstücks 22 einer Endoskopvorrichtung 10 gemäß Figur 1. Das distale Endstück 22 wird beispielhaft im Wesentlichen von dem distalen Abschnitt 16 des Schafts 12 gebildet. Insofern kann sich der proximale Schaftabschnitt 14 bis zum distalen Endstück 22 erstrecken. Alternativ kann der distale Schaftabschnitt 16 denjenigen Teil des Schafts 12 umfassen, der zum Einführen vorgesehen ist. Der proximale Schaftabschnitt 14 kann dann lediglich derjenige Abschnitt des Schafts 12 sein, der im eingeführten Zustand des Schafts 12 außerhalb des Patienten verbleibt. In der gezeigten Ausführungsform ist ein Außengehäuse des distalen Endstücks 22 als ein Abschnitt eines Hohlzylinders ausgebildet. Das Endstück 22 ist ferner mit einem proximalen Abschnitt 14 des Schafts 12 über Kopplungsbereiche 98a,b funktional und verliersicher koppelbar. So kann das Endstück beispielsweise magnetisch oder über eine komplementäre Gewindevorrichtung reversibel an dem proximalen Abschnitt 14 des Schafts 12 montiert werden. Alternativ kann der Endabschnitt 22 aber auch einstückig mit dem proximalen Schaftabschnitt 14 ausgebildet sein. Eine derartige Ausgestaltung vereinfacht insbesondere die Herstellung, Wartung und bei Bedarf das Auswechseln einer Endoskopvorrichtung 10.

Am äußerst distalen Ende des Endstücks 22, schließt eine Endfläche 100 die Öffnung des Hohlzylinders formschlüssig. Die Endfläche 100 kann beispielsweise auf die Öffnung aufgeschraubt, fest mit dieser verklebt oder einstückig mit dem Hohlzylinder ausgebildet sein. In der in Figur 2 gezeigten Ausführungsform ist in der Endfläche 100 ferner eine Aussparung vorgesehen, in und/oder hinter der eine Eingangsoptik 30 angeordnet ist. In der gezeigten Ausführungsform ist in der in der Endfläche 100 ausgebildeten Aussparung ein Schutzglas 108 montiert, das die Bilderfassungsbaugruppen vor äußeren Einflüssen schützt, ohne die spektralen Eigenschaften von Abbildungslicht AL eines abzubildenden Objekts 21 zu beeinflussen. In einer alternativen Ausführungsform kann auch die Eingangsoptik 30 formschlüssig in der Aussparung angeordnet sein. Das Schutzglas 108 kann außerdem auch als Teil der Eingangsoptik 30 aufgefasst werden.

Dabei erstreckt sich die Eingangsoptik 30 parallel zu der Längsachse LA des distalen Abschnitts 16 in das Innere des Hohlzylinders und somit weg von der Endfläche 100. Die Eingangsoptik 30 ist dazu eingerichtet, das Abbildungslicht AL eines abzubildenden Objekts 21 in die Endoskopvorrichtung einzukoppeln. In anderen hier nicht gezeigten Ausführungsformen kann die Eingangsoptik 30 auch beispielsweise in der Mantelfläche des Schafts bzw. des Hohlzylinders angeordnet sein. Die Eingangsoptik 30 kann mehrere optische Elemente, beispielsweise in Form von verschiedenen Linsen, umfassen, die das Abbildungslicht fokussieren. Die Eingangsoptik 30 kann ein Objektiv sein. Dem Strahlenverlauf der Abbildungsstrahlen AL folgend, ist räumlich betrachtet hinter der Eingangsoptik 30 ein Strahlteiler 32 vorgesehen, der dazu eingerichtet ist, einen ersten Anteil des einfallenden Abbildungslichts HL der hyperspektralen Bilderfassungsbaugruppe 18 zuzuführen und einen zweiten Anteil einfallenden Lichts SL einer Kamerabaugruppe 24 der Endoskopvorrichtung 10 zuzuführen. Bei dem gezeigten Strahlteiler 32 kann es sich insbesondere um einen dichroitischen Strahlteiler handeln, der wellenlängenselektiv ist. Der Strahlteiler 32 ist insbesondere dazu eingerichtet, der Kamerabaugruppe 24 sichtbares Licht SL, insbesondere zumindest in einem Wellenlängenbereich von 450 nm bis 650 nm und vorzugsweise zumindest in einem Wellenlängenbereich von 420 nm bis 700 nm, zuzuführen und der hyperspektralen Bilderfassungsbaugruppe Infrarotlicht zuzuführen, insbesondere zumindest Licht mit einer Wellenlänge von über 1500 nm, bevorzugt Licht mit einer Wellenlänge von über 1000 nm und besonders bevorzugt Licht mit einer Wellenlänge von über 800 nm.

Hinter dem Strahlteiler 32 befindet sich eine der Kamerabaugruppe 24 zugehörige erste Bilderfassungssensorik 26a mit einem Bildsensor 28, der dazu eingerichtet ist, das durch den Strahlteiler transmittierte Licht zu detektieren. Insbesondere ist die erste Bilderfassungssensorik 26a dazu eingerichtet, Licht im sichtbaren Wellenlängenbereich zu detektieren.

Räumlich betrachtet, ist unter dem Strahlteiler 32, ein Spiegel 96 angeordnet, der das vom Strahlteiler 32 reflektierte Licht HL in Richtung der hyperspektralen Bilderfassungsbaugruppe 18 lenkt. Die hyperspektrale Bilderfassungsbaugruppe 18 umfasst eine Blende 34, die als eine Lochblende 40 ausgestaltet ist. Diese Blende 34 ist dazu eingerichtet, das vom Spiegel 96 in Richtung der hyperspektralen Bilderfassungsbaugruppe 18 umgelenkte Licht HS gemäß einem Blendenmuster auszublenden und durchzulassen. Hinter der Blende 34 befindet sich eine Aufspaltungsoptik 36, die dazu eingerichtet ist, durch die Blende 34 durchgelassenes Licht HS entlang einer Raumachse spektral aufzuspalten. Auf die Aufspaltungsoptik 36 folgt eine fokussierende Optik 102 in Form einer Kollimationslinse, die den Strahlenverlauf des spektral aufgespaltenen Lichts auf einen lichtempfindlichen Bereich 38 einer zweiten der hyperspektralen Bilderfassungsbaugruppe 18 zugehörigen Bilderfassungssensorik 26b lenkt. Wie auch die Eingangsoptik 30, kann auch die fokussierende Optik 102 mehrere optische Elemente beispielsweise in Form von verschiedenen Linsen umfassen.

Die in Figur 2 gezeigte hyperspektrale Bilderfassungsbaugruppe 18 umfasst außerdem eine Blendenverlagerungseinheit 44, die einen Aktor 46 und einen Blendenträger 48 umfasst. Der Aktor 46 ist in dieser Ausführungsform als Piezoaktor 52 konfiguriert. Die Blendenverlagerungseinheit 44 ist dazu eingerichtet, die Blende 34 relativ zu der Bilderfassungssensorik 26b zu bewegen. Der Blendenträger 48 ist sowohl mit dem Aktor 46 als auch mit der Blende 34 verbunden. Ferner umfasst der Blendenträger 48 ein Festkörpergelenk 50. Der Aktor 46 ist dazu eingerichtet, eine Linearbewegung zu erzeugen. Diese Linearbewegung wird mittels des Festkörpergelenks 50 in eine Schwenkbewegung der Blende 34 umgewandelt. Der Bewegungsverlauf des Festkörpergelenks 50 sowie die daraus resultierende Bewegung der Blende 34 ist durch die beiden Doppelpfeile angedeutet. Der Bewegungsverlauf des Festkörperelements 50 und der daraus resultierenden Schwenkbewegung der Blende 34 ist durch zwei Pfeile angedeutet.

Der Blendenträger 48 weist eine Länge auf, welche den Schaftdurchmesser übersteigt. Die Länge kann beispielsweise wenigstens dem 1,5-fachen, wenigstens dem 2-fachen oder wenigstens dem 3-fachen des Schaftdurchmessers entsprechen. Hierdurch werden bereits kleine Schwenkbewegungen im Bereich des Festkörpergelenks 50 in Seitwärtsbewegungen der Blende 34 übersetzt, die sich über wenigstens 10%, wenigstens 20% oder sogar wenigstens 30% des Schaftdurchmessers erstrecken. Zudem ist die Bewegung der Blende aufgrund der Ausdehnung des Blendenträgers 48 näherungsweise linear. Das Festkörpergelenk 50 ist hierdurch nur geringfügigen Belastungen ausgesetzt, da es Rotationen nur um einige Grad aufnehmen muss, beispielsweise um weniger als 20 Grad, um weniger als 10 Grad oder sogar um weniger als 5 Grad.

Bezüglich der Längsachse LA des distalen Abschnitts 16 ist der Aktor 46 von der Eingangsoptik 30 aus betrachtet hinter der Bilderfassungssensorik 26b angeordnet, sodass der Blendenträger 48 die Bilderfassungssensorik 26b überspannt.

In dieser Ausführungsform sind bezüglich der Längsachse LA des distalen Abschnitts 16 des Schafts 12 die Kamerabaugruppe 24 und die hyperspektrale Bilderfassungsbaugruppe 18 nebeneinander angeordnet. Gemäß dieser Anordnung verlaufen der Strahlengang von dem Strahlenteiler 32 zu der Bilderfassungssensorik 26a der Kamerabaugruppe und der Strahlengang von dem Strahlteiler 32 zu der Bilderfassungssensorik 26b der hyperspektralen Bilderfassungsbaugruppe 18 zumindest abschnittsweise parallel zueinander. Durch die überspannende Anordnung kann die erforderliche Baulänge des Blendenträgers 48 erreicht werden, ohne durch die Blende 34 hindurchtretendes Licht über eine unnötig weite Strecke führen zu müssen. Zudem kann hierdurch der Aktor 46 auf einer der Eingangsoptik 30 abgewandten Seite der Bilderfassungssensorik angeordnet sein, sodass dieser mit dem Lichtweg der Bilderfassungsbaugruppe 18 nicht wechselwirkt.

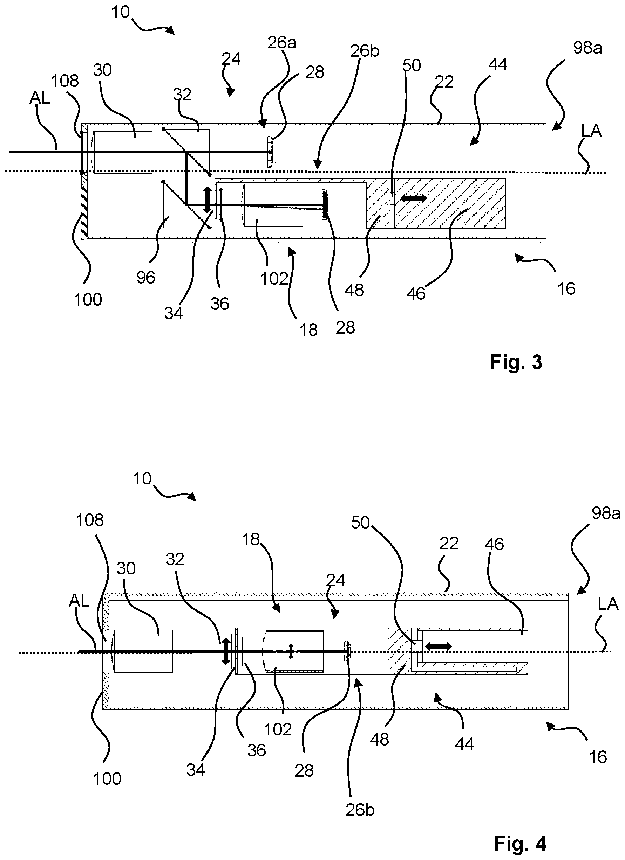

Die Figuren 3 und 4 zeigen jeweils eine andere Ansicht des distalen Endstücks 22 der Endoskopvorrichtung 10 gemäß Figur 2. Figur 3 zeigt eine schematische Querschnittsansicht des distalen Endstücks 22 und Figur 4 eine schematische Draufsicht des distalen Endstücks 22.

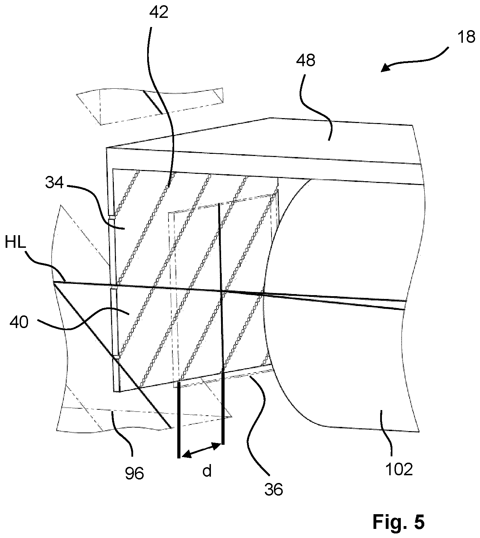

Figur 5 stellt eine perspektivische Detailansicht eines Teilabschnitts der hyperspektralen Bilderfassungsbaugruppe gemäß Figur 2 dar. Die mit dem Blendenträger 48 verbundene Blende umfasst mehrere Reihen 116 von Blendenlöchern 42, die bezüglich einer Querrichtung der Aufspaltungsoptik 36 parallel verlaufen. Ferner sind die Reihen 116 von Blendenlöchern 42 um einen Lochreihenabstand d voneinander beabstandet. An dieser Stelle wird darauf hingewiesen, dass das in dieser Ausführungsform gezeigte Blendenmuster lediglich beispielhaft ist. Es versteht sich, dass das in dieser Figur gezeigte Blendenmuster und oder auch die Blendenform je nach Ausführungsform und Anwendungsbereich variieren kann. Insbesondere können in anderen Ausführungsformen die Anzahl der Blendenlöcher 42, die Anzahl der Reihen 116 der Blendenlöcher 42, der Lochreihenabstand d, die Größe der Blendenlöcher 42, die Form der Blendenlöcher 42 sowie die Anordnung der Blendenlöcher 42 auf der Blende 34 variieren. Selbiges gilt im Übrigen auch für alle nachfolgend beschriebenen Blende.

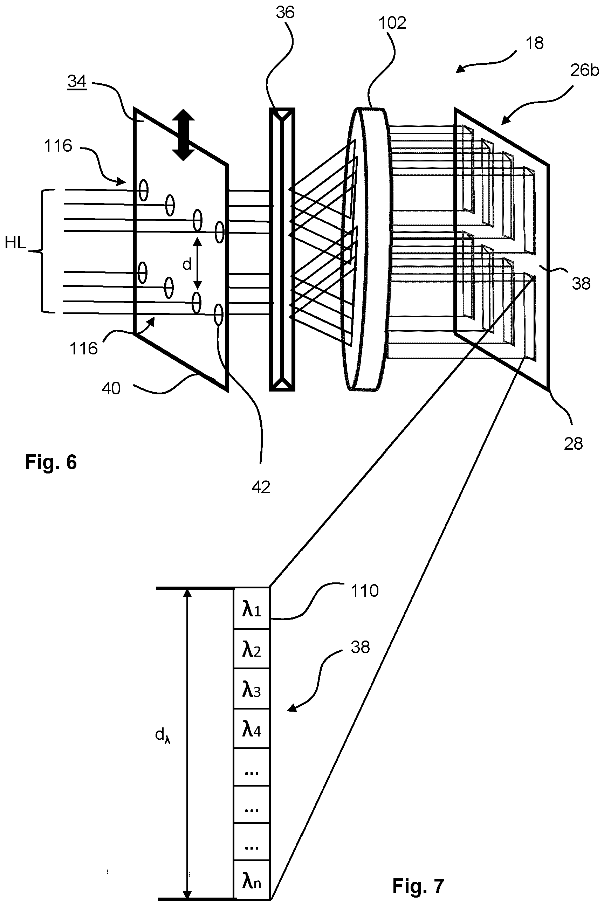

Figur 6 zeigt eine vereinfachte Darstellung der hyperspektralen Bilderfassungsbaugruppe 18 gemäß den Figuren 2 bis 3. Durch die Lochblende 40 wird Licht HL gemäß eines durch die Blendenlöcher 42 definierten Blendenmusters ausgeblendet und durchgelassen. Der Übersichtlichkeit halber ist lediglich ein Blendenloch 42 mit einem Bezugszeichen versehen. Das durchgelassene Licht trifft hinter der Lochblende 40 auf eine Aufspaltungsoptik 36, die dazu eingerichtet ist, das Licht in einem von der hyperspektralen Bilderfassungsbaugruppe vordefinierten Wellenlängenbereich entlang einer räumlichen Achse über einen Aufspaltungsabstand d_{λ} spektral aufzuspalten. Dabei ist der Aufspaltungsabstand höchstens so groß wie der Lochreihenabstand d. Vorzugsweise ist er etwas kleiner als ein entlang des Strahlengangs auf den Bildsensor 28 projizierter Lochreihenabstand wäre, beispielsweise um wenigstens 10% oder wenigstens 20% kleiner, damit sich keine Überlappungen auf dem Bildsensor 28 ergeben. Hinter der Aufspaltungsoptik ist eine fokussierende Optik 102 angeordnet, die den Strahlenverlauf des aufgespaltenen Lichts auf einen lichtempfindlichen Bereich 38 des Bildsensors 28 der Bilderfassungssensorik 26b lenkt. Die Blendenverlagerungseinheit 44 ist in dieser Darstellung nicht gezeigt, die Bewegungsrichtung der Blende jedoch schematisch durch einen Doppelpfeil illustriert.

Figur 7 zeigt beispielhaft eine schematische Darstellung eines in Figur 6 angedeuteten Teilbereichs des lichtempfindlichen Bereichs 38, auf den spektral aufgespaltenes Licht fällt, das von einem einzelnen Blendenloch herrührt. Der lichtempfindliche Bereich umfasst eine Matrix von Bildpixeln, die dazu eingerichtet sind, Wellenlängenkomponenten des zuvor spektral aufgespaltenen Lichts aufzunehmen und in elektrische Signale umzuwandeln. Vorliegend ist der Bildsensor 28 beispielsweise ein CCD- oder CMOS-Sensor, vorzugsweise ohne Farbfiltermatrix. Dieser detektiert somit Licht sämtlicher Wellenlängen des aufgespalteten Wellenlängenbereichs. Für die Zwecke der hyperspektralen Bilddatenerfassung wird der lichtempfindliche Bereich 38 in ein Detektionsarray aufgeteilt, das beispielhaft einzelne Bildzellen 110 umfassen kann. Jeder dieser Bildzellen 110 kann eine bestimmte Wellenlänge bzw. ein bestimmter Wellenlängenbereich zugeordnet sein. Die Aufteilung in Bildzellen 110 kann von den einzelnen Pixeln des Bildsensors 28 verschieden sein. Ein Streifen derartiger Bildzellen 110 kann dann zur Gewinnung diskreter spektraler Messpunkte für einen bestimmten räumlichen Punkt des erfassten Bildes verwendet werden, wobei besagter räumlicher Punkt durch das betreffende Blendenloch 42 definiert ist. Mit Kenntnis über den Aufspaltungsabstand d_{λ} können die elektrischen Signale unterschiedlicher Bildzellen 110 unterschiedlichen Wellenlängenbereiche λ1- n zugeordnet werden.

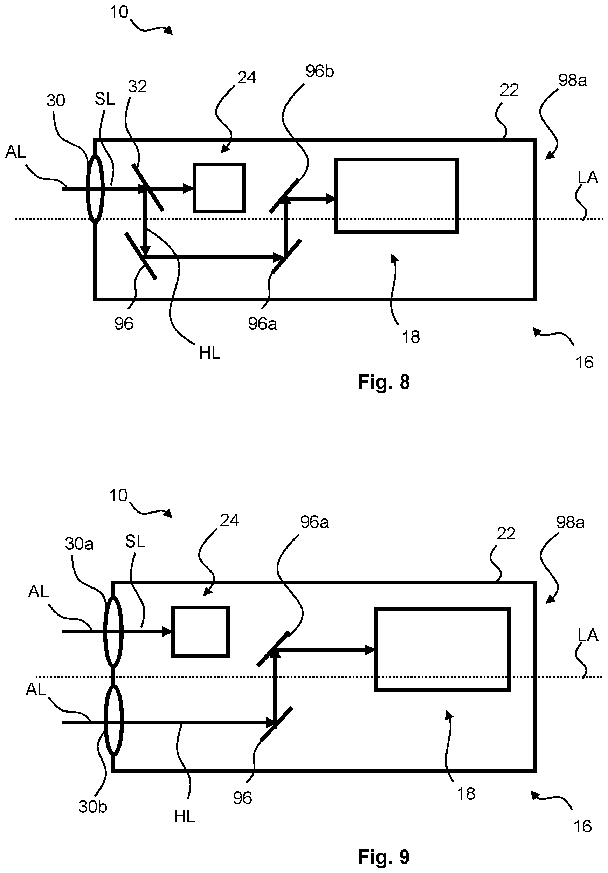

Die Figuren 8 und 9 zeigen weitere Ausführungsformen der Endoskopvorrichtung 10, bei denen die Kamerabaugruppe 24 und die hyperspektrale Bilderfassungsbaugruppe 18 nicht nebeneinander, sondern hintereinander, entlang der Längsachse LA des Endabschnitts 22 der Endoskopvorrichtung 10, angerordnet sind. Die Kamerabaugruppe 24 sowie die hyperspektrale Bilderfassungsbaugruppe 18 sind in den Figuren 8 und 9 vereinfacht als Blackbox dargestellt. Der detaillierte Aufbau ist den Figuren 2 und 3 zu entnehmen. Um die Kamerabaugruppe 24 sowie die hyperspektrale Bilderfassungsbaugruppe 18 hintereinander anordnen zu können, weist die Endoskopvorrichtung 10 zusätzliche Spiegel 96a,b auf. Diese Spiegel 96a,b sind dazu eingerichtet das Abbildungslicht AL bzw. Anteile dessen HL platzsparend umzulenken.

In der in Figur 9 gezeigten Ausführungsform, weist die Endoskopvorrichtung 10 neben der Eingangsoptik 30a eine zusätzliche Eingangsoptik 30b auf. Die zusätzliche Eingangsoptik 30b ist neben der Eingangsoptik 30a angeordnet. In einigen Ausführungsformen können die beiden Eingangsoptiken 30a,b baugleich oder unterschiedlich ausgebildet sein. Auch die Position und/oder die Anzahl weiterer Eingangsoptiken 30 kann in hier nicht gezeigten Ausführungsformen, je nach Anwendungsbereich, variieren. In der gezeigten Ausführungsform ist die erste Eingangsoptik 30a dazu eingerichtet Abbildungslicht einzukoppeln und zu der Bilderfassungssensorik 26a der Kamerabaugruppe 24 zu leiten. Die zusätzliche Eingangsoptik 30b dient dazu Abbildungslicht einzukoppeln und zu der Bilderfassungssensorik 26b der hyperspektralen Bilderfassungsbaugruppe zu lenken. Somit weisen die beiden Baugruppen 24, 18 gemäß dieser Ausführungsform keine gemeinsame Eingangsoptik 30 sondern separate Eingangsoptiken 30a,b auf.

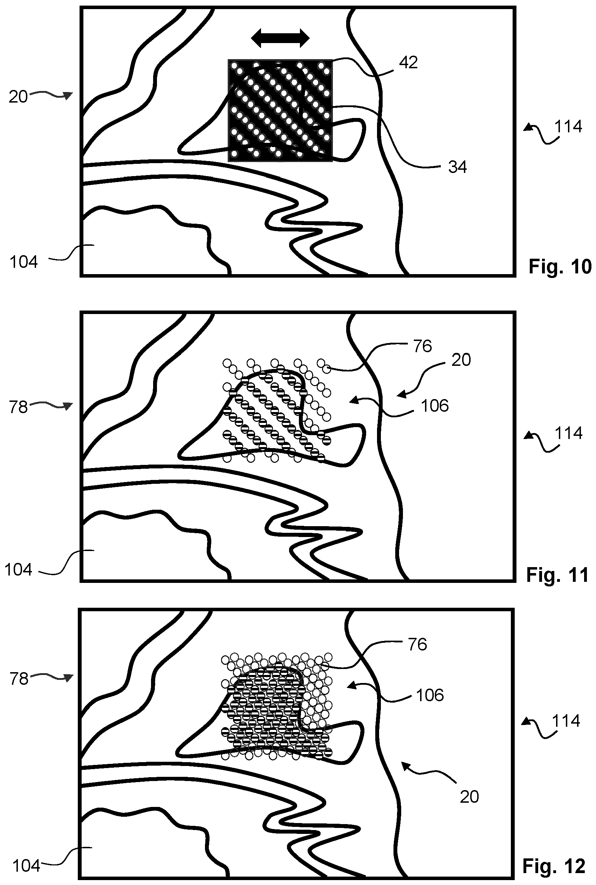

In den Figuren 10 bis 12 sind Teilschritte einer Bilderzeugung mit einer in Figur 2 dargestellten Endoskopvorrichtung 10 beispielhaft illustriert. Dargestellt ist ein Objektbereich 20 mit einer Vielzahl an Gewebestrukturen 104. Die Lochblende 40 bzw. deren Blendenlöcher 42 sind in einer Ausgangsposition dazu eingerichtet Bildpunkte 76 des abzubildenden Objektbereichs gemäß des Blendenmusters auszuwählen und deren Licht zu einer Bilderfassungssensorik 26b zu leiten, welche die spektrale Information der Bildpunkte 76 erfasst. Die für die Bildpunkte 76 gewonnene spektrale Information kann dann ausgewertet und beispielsweise einem Weißlichtbild überlagert werden. Der Doppelpfeil über der Lochblende 40 deutet den Freiheitsgrad bzw. die beiden möglichen Bewegungsrichtungen der Lochblende 40 an. Durch Bewegen der Lochblende 40 können nacheinander für unterschiedliche räumliche Punkte des Bildes Spektren erfasst werden.

Figur 11 zeigt eine beispielhafte Überlagerungsdarstellung 78. Einem Weißlichtbild 114 ist dabei in einem mittleren Bildbereich106 Bildinformation überlagert, die auf spektral aufgelösten Bilddaten beruht. Beispielhaft sind im gezeigten Fall unterschiedliche Gewebearten unterschiedlich hervorgehoben, wobei die Gewebearten anhand derjenigen spektralen Information automatisiert identifiziert werden, die aus den oben beschriebenen unterschiedlichen Bildpunkten 76 gewinnbar ist. Der Übersichtlichkeit halber ist lediglich ein Bildpunkt 76 mit einem Bezugszeichen versehen. Da lediglich für den durch die Blende 34 ausgewählten Bildbereich 106 Spektraldaten vorhanden sind, ist entsprechend nur ein Teil der Überlagerungsdarstellung mit derartiger Information versehen. Vorliegend wird diese Information für einen mittleren Bereich gewonnen, was zweckmäßig sein kann, da ein Benutzer tendenziell die relevanten Objektbereiche mittig im Bild darstellen möchte. Obwohl spektrale Information lediglich abschnittsweise bzw. punktweise vorhanden ist, kann somit zusätzliche Information für die relevanten Bildbereiche 106 gewonnen werden.

Es versteht sich, dass alternativ andere Information dargestellt werden kann, die auf spektraler und räumlicher Information beruht, die für den Bildbereich 106 verfügbar ist. Beispielsweise kann ein Perfusionszustand oder ein anderer ortsaufgelöster Parameter eingeblendet werden, der auf einem Vergleich wenigstens zweier spektraler Intensitätswerte beruht, die unterschiedlichen Spektralbereiche und/oder unterschiedlichen Wellenlängen zugeordnet sind.

Ebenso kann vorgesehen sein, in dem Bildbereich 106 eine Intensitätsverteilung für eine bestimmte Wellenlänge und/oder einen bestimmten Wellenlängenbereich dem Weißlichtbild 114 zu überlagern. Hierdurch kann beispielsweise Fluoreszenz eines Farbstoffs sichtbar gemacht werden, der im entsprechenden Objektbereich eingebracht und in den spektral aufgelösten Bildpunkten 76 sichtbar ist.

Ferner versteht sich, dass anstelle eines Weißlichtbilds 114 auch ein anderes Bild die Grundlage für die Überlagerungsdarstellung 78 bilden kann, wie beispielsweise ein Fluoreszenzbild, ein monochromatisches Bild, ein Falschfarbenbild oder dergleichen.

Zudem kann ein Betriebsmodus vorgesehen sein, in dem lediglich der Bildbereich 106 dargestellt wird und/oder in dem im Bereich des Bildbereichs 106 lediglich Bilddaten angezeigt werden, die auf der spektralen Bilderfassung beruhen, also im Speziellen auf den Bildpunkten 76.

Durch eine mittels der Blendenverlagerungseinheit 44 herbeigeführten Verlagerung der Blende 42 relativ zu Bilderfassungssensorik 26b kann die räumliche Auflösung der spektral aufgelösten Bilddaten erhöht werden. In Figur 12 wurde die Blende 42 beispielhaft um einen Abstand nach rechts verlagert, der kleiner ist als der Lochreihenabstand d, sodass von der Blende 42 bei einer ersten Bildaufnahme verdecke Punkte betrachtet werden können, die nun in einem zweiten Bild durch die Blende 42 hindurch betrachtbar sind. In der verlagerten, von der Ausgangsposition abweichenden Position, werden von den Blendenlöchern 42 andere Bildpunkte 76 ausgewählt als in der in den Figuren 10 und 11 gezeigten Ausgangsposition. Im in der Figur 12 exemplarisch dargestellten Szenario erfasst die Blende 34 in der verlagerten Position, zumindest zu einem Großteil, den Bildbereich 106, der in der Ausgangsposition zwischen den Reihen 116 der Blendenlöcher 42 lag und dadurch in der Ausgangsposition nicht vermessen werden konnten.

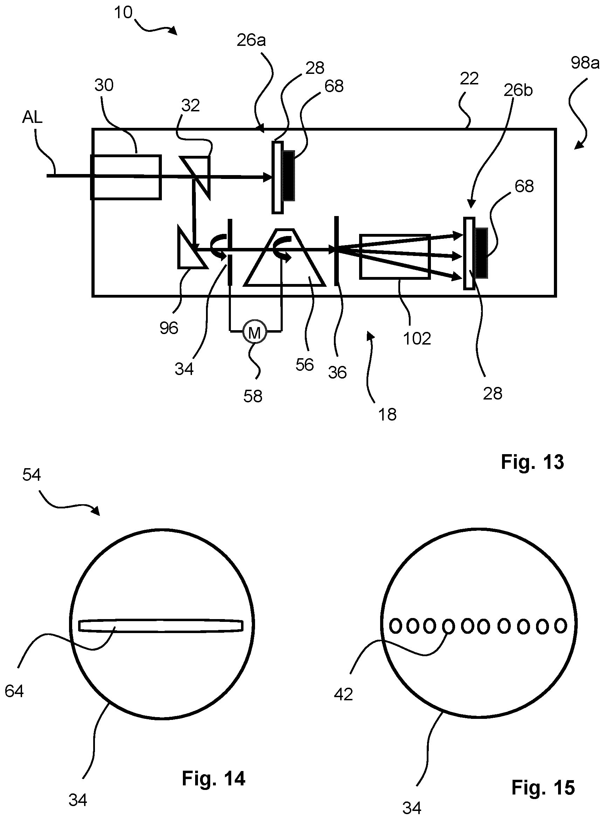

Figur 13 zeigt eine schematische Darstellung einer weiteren Ausführungsform eines distalen Endstücks 22 einer Endoskopvorrichtung 10. Diese Ausführungsform unterscheidet sich von den voranstehend beschriebenen im Wesentlichen im Aufbau der hyperspektralen Bilderfassungsbaugruppe 18. In dieser Ausführungsform ist die Blende 34 um eine Rotationsachse drehbar gelagert. Die Rotationsachse verläuft zumindest im Wesentlichen parallel zu der optischen Achse der Blende 34. Ferner ist die Blende 34 gemäß dieser Ausführungsform als eine Schlitzblende 54 ausgebildet. Es sei jedoch an dieser Stelle angemerkt, dass diese Ausführungsform keineswegs auf den Einsatz einer Schlitzblende 54 beschränkt ist. Wie den Ausführungen zu Figur 15 zu entnehmen ist, können auch andere, ebenfalls zur Rotation geeignete Blendenmuster vorgesehen sein. Hinter der Schlitzblende 54 ist ferner ein Dove-Prisma 56 angeordnet. Das Dove-Prisma 56 ist dazu eingerichtet, den Strahlenverlauf des Lichts des Bildbereichs 106, der durch die Schlitzblende 54 ausgewählt wird, umzulenken bzw. zu drehen, sodass das aus dem Dove-Prisma 56 wieder austretende Licht bzw. dessen Strahlverlauf nach und/oder während einer Rotation der Schlitzblende 54 um einen definierten Winkelbetrag stets die gleiche Orientierung in Bezug auf die Aufspaltungsoptik 36 hat. Darüber hinaus ist die Schlitzblende 54 mittels eines Drehantriebs 58 gemeinsam mit dem Dove-Prisma 56 drehbar gelagert. Der Drehantrieb 58 ist rein schematisch und nur bezogen auf seine Funktion dargestellt. Der Übersichtlichkeit halber ist es am unteren Rand der Fig. 13 eingezeichnet. Es versteht sich aber, dass der Drehantrieb 58 baulich ebenfalls innerhalb eines Gehäuses des distalen Endstücks 22 angeordnet ist.

In Figur 13 beispielhaft gezeigt, jedoch nicht auf den Einsatz in der darin dargestellten Ausführungsform beschränkt, sind Kühlvorrichtungen 68, die an der Endoskopvorrichtung 10, insbesondere an der Bilderfassungssensorik 26a,b, angeordnet sind. Diese Kühlvorrichtungen sind dazu eingerichtet, die Bilderfassungssensorik 26a,b während und/oder nach einem Betrieb zu kühlen. Hierbei kann es sich beispielsweise um Peltier-Kühlelemente, um Heatpipes, um kühlflüssigkeitsgespeiste Kühler und dergleichen handeln.

In den Figuren 14 und 15 sind beispielhaft zwei Blenden 34 gezeigt, die für den Einsatz in der Figur 13 gezeigten Ausführungsform einer hyperspektralen Bilderfassungsbaugruppe 18 geeignet sind. Diese beiden Blenden 34 unterscheiden sich in der Art des Blendenmusters. Figur 14 zeigt eine Schlitzblende und Figur 15 eine Blende, in der statt des Schlitzes eine Reihe 116 mit mehreren Blendenlöchern 42 vorgesehen ist. Aufgrund der Vielzahl an einsetzbaren Blenden 34, wird der Übersichtlichkeit halber, auf die Darstellung weiterer Blendenmuster verzichtet.

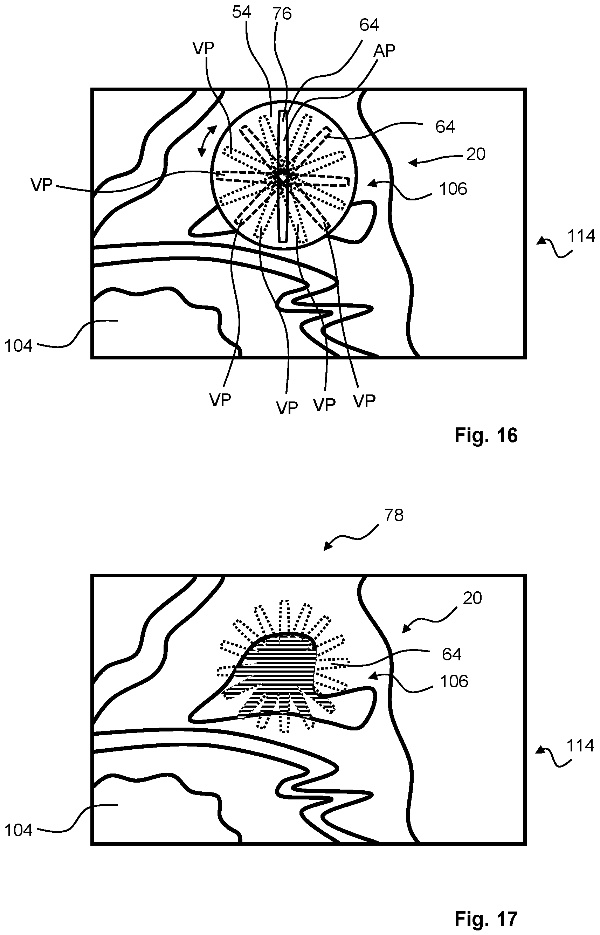

In den Figuren 16 und 17 sind Teilschritte einer Bilderzeugung mit einer in Figur 13 dargestellten Endoskopvorrichtung 10 beispielhaft illustriert. Dargestellt ist ein Objektbereich 20 mit einer Vielzahl an Gewebestrukturen 104. Die Schlitzblende 54 bzw. dessen Beobachtungsspalt 64 ist in einer Ausgangsposition AP sowie in mehreren um einen Winkelbetrag verdrehten Verlagerungspositionen VP gezeigt. Relativ zueinander verlagerte Beobachtungsspalte 64 sind in Fig. 16 mit durchgezogener Linie, gepunkteter Linie und gestrichelter Linie dargestellt.

Die Blende 34 ist dazu eingerichtet Bildstreifen 76 des abzubildenden Objektbereichs gemäß dem Beobachtungsspalt 64 auszuwählen und deren Licht zu einer Bilderfassungssensorik 26b zu leiten, welche die spektrale Information der Bildstreifen 76 erfasst. Die für die Bildstreifen 76 gewonnene spektrale Information kann dann ausgewertet und beispielsweise einem Weißlicht Bild überlagert werden.

Figur 17 zeigt eine beispielhafte Überlagerungsdarstellung 78. Einem Weißlichtbild 114 ist dabei in einem mittleren Bildbereich 106 Bildinformation überlagert, die auf spektral aufgelösten Bilddaten beruht. Beispielhaft sind im gezeigten Fall unterschiedliche Gewebearten unterschiedlich hervorgehoben, wobei die Gewebearten anhand derjenigen spektralen Information automatisiert identifiziert werden, die aus den oben beschriebenen unterschiedlichen Bildstreifen 76 gewinnbar ist. Da lediglich für den Bildbereich 106 Spektraldaten vorhanden sind, ist entsprechend nur ein Teil der Überlagerungsdarstellung 78 mit derartiger Information versehen.

Durch die mittels des Drehantriebs 58 herbeigeführte Verlagerung der Schlitzblende 54, relativ zu Bilderfassungssensorik 26b kann die räumliche Auflösung der spektral aufgelösten Bilddaten erhöht werden. In Figur 16 wurde die Schlitzblende 54 beispielhaft um definierte Winkelbeträge verlagert. In der verlagerten, von der Ausgangsposition abweichenden Position, werden von dem Beobachtungsspalt 64 andere Bildstreifen 76 ausgewählt als in der Ausgangsposition, die lediglich in einem Punkt in unmittelbarer Nähe um die Rotationsachse identisch sind. Vorteilhafterweise erhöht sich beim Drehen der Schlitzblende 54 beziehungsweise beim Drehen des Beobachtungsspalts 64 die räumliche Bildauflösung in einem mittleren Bereich, was zweckmäßig sein kann, da ein Benutzer eines Endoskops des relevanten Objektbereich 20 während einer therapeutischen und/oder diagnostischen Aktion bevorzugt mittig im Bild anvisiert und darstellen möchte.

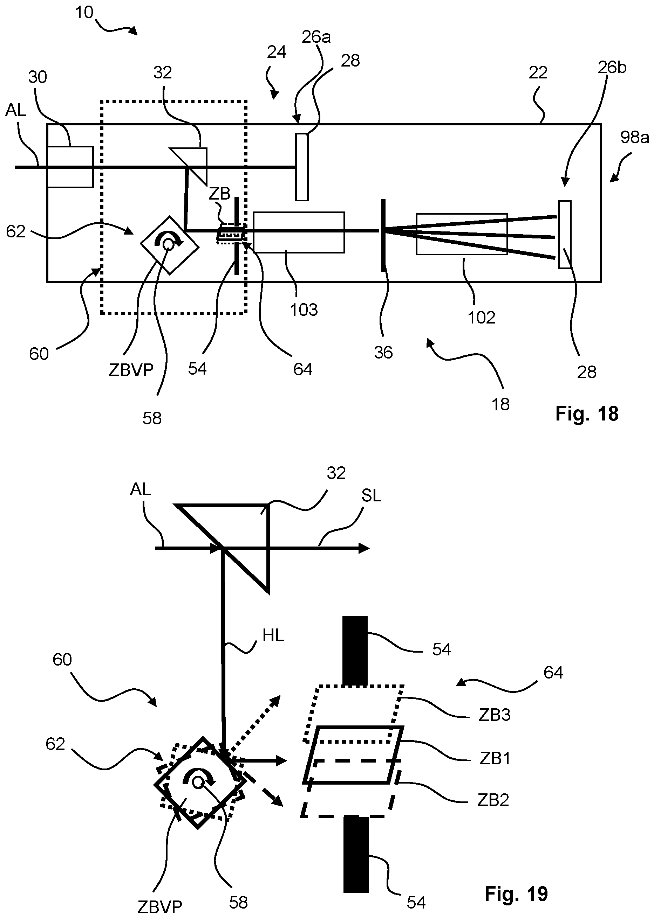

Figur 18 stellt eine schematische Darstellung einer weiteren Ausführungsform eines distalen Endstücks 22 einer Endoskopvorrichtung 10 dar. Die Endoskopvorrichtung 10 umfasst eine Eingangsoptik 30, die ein Zwischenbild ZB eines abzubildenden Objektbereichs 20 erzeugt. Ferner umfasst die Endoskopvorrichtung 10 eine Schlitzblende 54 mit einem Beobachtungsspalt 64, der einen Bildstreifen 76 des Zwischenbilds ZB auswählt und auf den das Zwischenbild ZB fällt.

Die in Figur 18 gezeigte Endoskopvorrichtung 10 umfasst außerdem eine optische
Zwischenbildverlagerungseinheit 60, die dazu eingerichtet ist, das Zwischenbild ZB relativ zum Beobachtungsspalt 64 optisch zu verlagern und dadurch unterschiedliche Bildstreifen 76 zu erzeugen. Relativ zueinander verlagerte Zwischenbilder ZB1, ZB2, ZB3 sind in Figur 18 und Figur 19 mit durchgezogener Linie, gepunkteter Linie und gestrichelter Linie dargestellt.

Erkennbar wird aus jedem dieser Zwischenbilder jeweils ein anderer Bildstreifen 76 durch den 10 Beobachtungsspalt 64 ausgewählt. Um das Zwischenbild ZB optisch verlagern zu können, umfasst die Zwischenbildverlagerungseinheit 60 ein optisches Element 62, das in der hier dargestellten Ausführungsform ein vierseitiges Prisma ZBVP ist. Das Prisma ZBVP ist mittels eines Drehantriebs 58 drehmomentübertragend gekoppelt. Das Prisma ZBVP kann durch Ansteuerung des Drehantriebs 58 um einen definierten Winkelbetrag verschwenkt werden. Durch Ansteuerung des Drehantriebs 58 kann die Zwischenbildverlagerungseinheit 60, wie in Figur 19 zu sehen, in unterschiedliche Positionen geschwenkt werden. Der Drehantrieb 58 ist insbesondere dazu eingerichtet ist, das optische Element 62 in eine vorgegebene Drehrichtung um insbesondere wenigstens 90 Grad und vorzugsweise zumindest 360 Grad und besonders bevorzugt kontinuierlich oder oszillatorisch zu drehen. Mit Drehung des optischen Elements ändert sich auch die Position des Zwischenbilds ZB. Der Beobachtungsspalt 64, das dispersives Element 36, die Bilderfassungssensorik 26b, die optische Zwischenbildverlagerungseinheit 60 als auch der Drehantrieb 58 sind Bestandteile der Endoskopvorrichtung 10 beziehungsweise der hyperspektralen Bilderfassungsbaugruppe 18. Hinter dem Beobachtungsspalt 64 ist eine Kollimatorlinse 103 angeordnet, die dazu eingerichtet ist, den Strahlenverlauf des einfallenden Lichts des mit dem Beobachtungsspalt 64 ausgewählten Bildstreifens 76 zu parallelisieren. Hinter der Kollimatorlinse 103 ist eine Aufspaltungsoptik 36 angeordnet, die dazu eingerichtet ist, das Licht des Bildstreifens entlang einer Raumachse spektral aufzuspalten. Auf die Aufspaltungsoptik 36 folgt eine fokussierende Optik, die den Strahlenverlauf des Bildstreifens 76 auf einen lichtempfindlichen Bereich 38 der Bilderfassungssensorik 26b lenkt. Anders als in den voranstehend beschriebenen Ausführungsformen wird gemäß der in Figur 18 gezeigten Ausführungsform die Schlitzblende 54 relativ zur Bildferfassungssensorik 26b nicht verlagert.

Figur 19 zeigt eine schematische Detailansicht einer optischen Zwischenbildverlagerungseinheit 60 gemäß Figur 18.

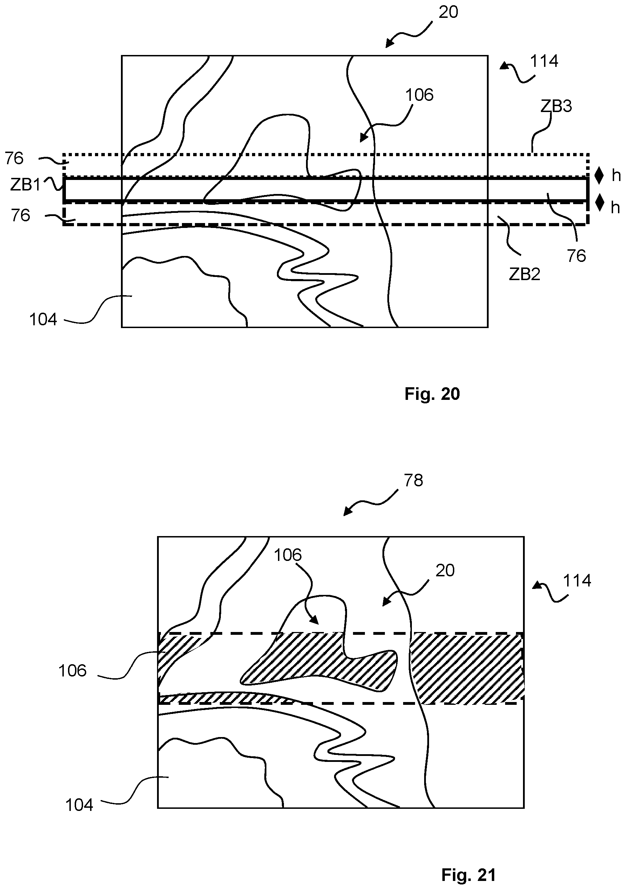

Figur 20 zeigt schematisch die Veränderung des Bildstreifens 76 in Folge einer Verlagerung des Zwischenbilds ZB1 bis ZB3 in Folge einer in Figur 19 gezeigten Zwischenbildverlagerung, herbeigeführt durch die optische Zwischenverlagerungseinheit 60. Dargestellt ist, ein Objektbereich 20 mit einer Vielzahl an Gewebestrukturen 104. Das Zwischenbild ZB1 stellt den Bildstreifen 76 dar, der von dem Beobachtungsspalt 64 der Schlitzblende 54 ausgewählt wird, wenn sich die Zwischenbildverlagerungseinheit 60 in der Ausgangsposition befindet. Durch eine Rotation der Zwischenverlagerungseinheit 60 verschiebt sich das Zwischenbild ZB2, ZB3 durch den abweichenden Strahlenverlauf in der Abbildung.Die beiden Zwischenbilder ZB2 und ZB3 liefern jeweils einen anderen Bildstreifen 76, dargestellt mit gepunkteten bzw. gestrichelten Linien.

Die für diese Bildstreifen 76 gewonnene spektrale Information kann dann ausgewertet und einem Weißlichtbild 114 überlagert werden. Fig. 21 zeigt eine beispielhafte Überlagerungsdarstellung 78. Einem Weißlichtbild 114 ist dabei in einem mittleren Bildbereich 106 Bildinformation überlagert, die auf spektral aufgelösten Bilddaten beruht. Beispielhaft sind im gezeigten Fall unterschiedliche Gewebearten 104 unterschiedlich hervorgehoben, wobei die Gewebearten anhand derjenigen spektralen Information automatisiert identifiziert werden, die aus den oben beschriebenen unterschiedlichen Bildstreifen 76 gewinnbar ist. Da lediglich für den Bildbereich 106 Spektraldaten vorhanden sind, ist entsprechend nur ein Teil der Überlagerungsdarstellung 78 mit derartiger Information versehen.

In weiteren Ausführungsformen kann auch eine Verlagerung des Zwischenbilds über dessen gesamte Höhe erfolgen, sodass Spektralinformation für das gesamte Bild gewinnbar ist.

Nachfolgend werden Aspekte beschrieben, die zum Verständnis der Erfindung beitragen:
Aspekt 1. Endoskopvorrichtung (10), umfassend:
   einen Schaft (12), der einen proximalen Abschnitt (14) und einen distalen Abschnitt (16) aufweist; und
   eine hyperspektrale Bilderfassungsbaugruppe (18), die dazu eingerichtet ist, Bilder eines Objektbereichs (20) eines abzubildenden Objekts (21) zu erfassen und hyperspektrale Bilddaten zu erzeugen, die räumliche und spektrale Information umfassen, wobei die Bilderfassungsbaugruppe (18) in dem distalen Abschnitt (16) angeordnet ist.
Aspekt 2. Endoskopvorrichtung (10) nach Anspruch 1,
   wobei der distale Abschnitt (16) ein distales Endstück (22) umfasst und wobei die hyperspektrale Bilderfassungsbaugruppe (18) in dem distalen Endstück (22) angeordnet ist.
Aspekt 3. Endoskopvorrichtung (10) nach Anspruch 1 oder 2,
   ferner umfassend eine Kamerabaugruppe (24), die wenigstens eine Bilderfassungssensorik (26a) mit wenigstens einem Bildsensor (28) umfasst, der dazu eingerichtet ist, Bilder zu erfassen und Bilddaten zu erzeugen.
Aspekt 4. Endoskopvorrichtung (10) nach Anspruch 3,
   wobei der Bildsensor (28) ein Farbbildsensor ist.
Aspekt 5. Endoskopvorrichtung (10) nach einem der vorherigen Ansprüche,
   ferner umfassend wenigstens eine Eingangsoptik (30), durch die Abbildungslicht (AL) einkoppelbar ist.
Aspekt 6. Endoskopvorrichtung (10) nach einem der Ansprüche 3 bis 5,
   ferner umfassend einen Strahlteiler (32), der hinter der Eingangsoptik (30) angeordnet ist;
   wobei der Strahlteiler (32) dazu eingerichtet ist, einen ersten Anteil einfallenden Lichts (HL) der hyperspektralen Bilderfassungsbaugruppe (18) zuzuführen und einen zweiten Anteil einfallenden Lichts (SL) der Kamerabaugruppe (24) zuzuführen.
Aspekt 7. Endoskopvorrichtung (10) nach Anspruch 6,
   wobei der Strahlteiler (32) wellenlängenselektiv ist, und wobei der Strahlteiler (32) dazu eingerichtet ist, der Kamerabaugruppe (24) sichtbares Licht, insbesondere zumindest in einem Wellenlängenbereich von 450 nm bis 650 nm und vorzugsweise zumindest in einem Wellenlängenbereich von 420 nm bis 700 nm, zuzuführen und der hyperspektralen Bilderfassungsbaugruppe Infrarotlicht zuzuführen, insbesondere zumindest Licht mit einer Wellenlänge von über 1500 nm, bevorzugt Licht mit einer Wellenlänge von über 1000 nm und besonders bevorzugt Licht mit einer Wellenlänge von über 800 nm.
Aspekt 8. Endoskopvorrichtung (10) nach einem der Ansprüche 1 bis 7,
   wobei die hyperspektrale Bilderfassungsbaugruppe (18) umfasst:
      eine Blende (34), die dazu eingerichtet ist, das Licht (HL, SL) gemäß einem Blendenmuster auszublenden und durchzulassen;
      eine Aufspaltungsoptik (36), die dazu eingerichtet ist, durch die Blende (34) durchgelassenes Licht (HL, SL) spektral aufzuspalten; und wenigstens
      eine zweite Bilderfassungssensorik (26b) mit wenigstens einem Bildsensor (28), wobei die Bilderfassungssensorik (26b) einen lichtempfindlichen Bereich (38) definiert und bezüglich der Aufspaltungsoptik (36) derart angeordnet ist, dass spektral aufgespaltetes Licht auf den lichtempfindlichen Bereich (38) fällt;
   wobei die Eingangsoptik (30), die Blende (34), die Aufspaltungsoptik (36) und die zweite Bilderfassungssensorik (26b) in dem distalen Abschnitt (16) angeordnet sind.
Aspekt 9. Endoskopvorrichtung (10) nach Anspruch 8,
   wobei ein Strahlengang von dem Strahlteiler (32) zu der Bilderfassungssensorik (26a) der Kamerabaugruppe (24) und ein Strahlengang von dem Strahlteiler (32) zu der Bilderfassungssensorik (26b) der hyperspektralen Bilderfassungsbaugruppe (18) zumindest abschnittsweise parallel zueinander verlaufen.
Aspekt 10. Endoskopvorrichtung (10) nach einem der Ansprüche 3 bis 9,
   wobei bezüglich einer Längsachse des distalen Abschnitts (16) des Schafts (12) die Kamerabaugruppe (24) und die hyperspektrale Bilderfassungsbaugruppe (18) nebeneinander angeordnet sind.
Aspekt 11. Endoskopvorrichtung (10) nach einem der Ansprüche 3 bis 10,
   wobei bezüglich einer Längsachse des distalen Abschnitts (16) des Schafts (12) die Kamerabaugruppe (24) und die hyperspektrale Bilderfassungsbaugruppe (18) hintereinander angeordnet sind.
Aspekt 12. Endoskopvorrichtung (10) nach einem der Ansprüche 3 bis 11,
   wobei die erste und/oder zweite Bilderfassungssensorik (26a,b) relativ zu dem distalen Abschnitt (16) des Schafts (12) ortsfest ist.
Aspekt 13. Endoskopvorrichtung (10) nach einem der Ansprüche 8 bis 12,
   wobei die Eingangsoptik (30) und die Aufspaltungsoptik (36) relativ zu dem distalen Abschnitt (16) des Schafts (12) ortsfest sind.
Aspekt 14. Endoskopvorrichtung (10) nach einem der Ansprüche 8 bis 13,
   wobei die Blende (34) als eine Lochblende (40) ausgebildet ist, die mehrere Reihen von Blendenlöchern (42) umfasst.
Aspekt 15. Endoskopvorrichtung (10) nach Anspruch 14,
   wobei die Reihen von Blendenlöchern (42) bezüglich einer Querrichtung der Aufspaltungsoptik (36) und/oder des lichtempfindlichen Bereichs (38) schräg verlaufen.
Aspekt 16. Endoskopvorrichtung (10) nach Anspruch 14 oder 15,
   wobei Reihen von Blendenlöchern (42) um einen Lochreihenabstand (d) voneinander beabstandet sind, wobei die hyperspektrale Bilderfassungsbaugruppe (18) einen auflösbaren Wellenlängenbereich definiert, und wobei die Aufspaltungsoptik (36) dazu eingerichtet ist, den auflösbaren Wellenlängenbereich entlang einer räumlichen Achse über einen Aufspaltungsabstand (λ) aufzuspalten, der höchstens so groß ist wie der Lochreihenabstand (d).
Aspekt 17. Endoskopvorrichtung (10) nach einem der Ansprüche 8 bis 16,
   wobei die Blende (34) relativ zu der zweiten Bilderfassungssensorik (26b) bewegbar gelagert ist.
Aspekt 18. Endoskopvorrichtung (10) nach einem der Ansprüche 8 bis 17,
   wobei die Bilderfassungsbaugruppe (18) eine Blendenverlagerungseinheit (44) umfasst, die einen Aktor (46) und einen Blendenträger (48) umfasst, wobei der Blendenträger (48) sowohl mit dem Aktor (46) als auch mit der Blende (34) verbunden ist, und wobei mittels des Aktors (46) die Blende (34) relativ zu der zweiten Bilderfassungssensorik (26b) bewegbar ist.
Aspekt 19. Endoskopvorrichtung (10) nach Anspruch 18,
   wobei der Blendenträger (48) ein Festkörpergelenk (50) umfasst.
Aspekt 20. Endoskopvorrichtung (10) nach Anspruch 18 oder 19,
   wobei der Aktor (46) dazu eingerichtet ist, eine Linearbewegung zu erzeugen, und wobei mittels des Festkörpergelenks (50) die Linearbewegung in eine Schwenkbewegung der Blende (34) wandelbar ist.
Aspekt 21. Endoskopvorrichtung (10) nach einem der Ansprüche 18 bis 20,
   wobei der Aktor (46) ein Piezoaktor (52) ist.
Aspekt 22. Endoskopvorrichtung (10) nach einem der Ansprüche 18 bis 21,
   wobei bezüglich einer Längsachse (LA) des distalen Abschnitts (16) der Aktor (46) von der Eingangsoptik (30) aus betrachtet hinter der zweiten Bilderfassungssensorik (26b) angeordnet ist, und wobei der Blendenträger (48) die zweite Bilderfassungssensorik (26b) überspannt.
Aspekt 23. Endoskopvorrichtung (10) nach einem der Ansprüche 8 bis 13,
   wobei die Blende (34) um eine Rotationsachse drehbar gelagert ist, die zumindest im Wesentlichen parallel zu einer optischen Achse der Blende (34) angeordnet ist.
Aspekt 24. Endoskopvorrichtung (10) nach Anspruch 23,
   wobei die Blende (34) als eine Schlitzblende (54) ausgebildet ist.
Aspekt 25. Endoskopvorrichtung (10) nach Anspruch 23 oder 24,
   wobei die Bilderfassungsbaugruppe (18) ein Dove-Prisma (56) umfasst, das gemeinsam mit der Blende (34) drehbar gelagert ist.
Aspekt 26. Endoskopvorrichtung (10) nach einem der Ansprüche 23 bis 25,
   wobei die Bilderfassungsbaugruppe (18) einen Drehantrieb (58) umfasst, der dazu eingerichtet ist, die Blende (34) und/oder das Dove-Prisma (56) um die Rotationsachse zu drehen.
Aspekt 27. Endoskopvorrichtung (10) nach einem der Ansprüche 5 bis 26,
   wobei die Eingangsoptik (30) dazu eingerichtet ist, ein Zwischenbild (ZB) eines abzubildenden Objekts (21) zu erzeugen, das auf die Blende (34) fällt, sodass mittels eines Beobachtungsspalts (64) der Blende (34) ein Zwischenbildabschnitt des Zwischenbilds (ZBA) auswählbar ist; und
   wobei die hyperspektrale Bilderfassungseinheit (18) eine optische Zwischenbildverlagerungseinheit (60) umfasst, die dazu eingerichtet ist, das Zwischenbild (ZB) relativ zu der Blende (34) optisch zu verlagern, um unterschiedliche Zwischenbildabschnitte (ZBA) des Zwischenbilds (ZB) auszuwählen.
Aspekt 28. Endoskopvorrichtung (10) nach Anspruch 27,
   wobei die optische Zwischenbildverlagerungseinheit (60) ein bewegliches optisches Element (62) umfasst, das dazu eingerichtet ist, die Position des Zwischenbilds (ZB) relativ zu dem Beobachtungsspalt (64) zu verlagern.
Aspekt 29. Endoskopvorrichtung (10) nach Anspruch 28,
   wobei das optische Element (62) zwischen der Eingangsoptik (30) und der Blende (34) angeordnet ist.
Aspekt 30. Endoskopvorrichtung (10) nach Anspruch 28 oder 29,
   wobei das optische Element (62) ein Prisma (ZBVP), insbesondere ein wenigstens vierseitiges Prisma (ZBVP), umfasst.
Aspekt 31. Endoskopvorrichtung (10) nach einem der Ansprüche 28 bis 30,
   wobei die zweite Bilderfassungssensorik (26b) dazu eingerichtet ist, während einer kontinuierlichen Bewegung des optischen Elements (62) kontinuierlich hyperspektrale Bilddaten zu erfassen.
Aspekt 32. Endoskopvorrichtung (10) nach einem der Ansprüche 28 bis 31,
   wobei eine Datenerfassung der zweiten Bilderfassungssensorik (26b) mit der kontinuierlichen Bewegung des optischen Elements (62) synchronisiert ist.
Aspekt 33. Endoskopvorrichtung (10) nach einem der Ansprüche 28 bis 32,
   wobei die Zwischenbildverlagerungseinheit (60) einen Antrieb (58) für das optische Element (62) umfasst, der dazu eingerichtet ist, das optische Element (62) in eine vorgegebene Drehrichtung um insbesondere wenigstens 90 Grad und vorzugsweise zumindest 360 Grad oder besonders bevorzugt kontinuierlich zu drehen.
Aspekt 34. Endoskopvorrichtung (10) nach einem der Ansprüche 28 bis 33,
   wobei die Zwischenbildverlagerungseinheit (60) einen Pendelantrieb für das optische Element (62) umfasst, der dazu eingerichtet ist, das optische Element (62) oszillatorisch zu bewegen, insbesondere gemäß einer Pendelbewegung.
Aspekt 35. Endoskopvorrichtung (10) nach einem der Ansprüche 27 bis 34,
   wobei die Zwischenbildverlagerungseinheit dazu eingerichtet ist, das Zwischenbild relativ zu dem Beobachtungsspalt (64) der Blende (34) zu verlagern.
Aspekt 36. Endoskopvorrichtung (10) nach einem der Ansprüche 3 bis 35,
   ferner umfassend eine Kühlvorrichtung (68), die dazu eingerichtet ist, die erste und/oder zweite Bilderfassungssensorik (26a,b) zu kühlen.
Aspekt 37. Endoskop (70) mit einer Endoskopvorrichtung (10) nach einem der vorhergehenden Ansprüche.
Aspekt 38. Medizinisches System (72), umfassend:
   ein Endoskop (70) gemäß Anspruch 37; und
   eine Darstellungserzeugungseinheit (74), die dazu eingerichtet ist, eine Darstellung (78) für einen Benutzer zu erzeugen, die auf einem Zusammenfügen von Information unterschiedlicher Bildpunkte (76a) und/oder Bildstreifen (76b) zu einem Bildteilbereich beruht.
Aspekt 39. Medizinisches System (72) nach Anspruch 38,
   wobei die Darstellung (78) eine Überlagerung des Bildteilbereichs und eines Bilds des abzubildenden Objektbereichs (20) umfasst.
Aspekt 40. Medizinisches System (72) nach Anspruch 38 oder 39,
   ferner umfassend eine Analyseeinheit (80), die dazu eingerichtet ist, nach Maßgabe von Bilddaten der ersten und/oder zweiten Bilderfassungssensorik (26a,b) eine Analyse zu erstellen, die auf spektraler Information der Bildstreifen (76b) beruht.
Aspekt 41. Medizinisches System (72) nach Anspruch 40,
   wobei die Analyse die Ermittlung zumindest eines physiologischen Parameters, insbesondere eines Perfusionsparameters, umfasst.
Aspekt 42. Medizinisches System (72) nach Anspruch 40 oder 41,
   wobei die Darstellungserzeugungseinheit (74) dazu eingerichtet ist, eine Darstellung für einen Benutzer zu erzeugen, die auf der Analyse beruht.

### Bezugszeichenliste

- 10: Endoskopvorrichtung
- 12: Schaft
- 14: proximaler Schaftabschnitt
- 16: distaler Schaftabschnitt
- 18: hyperspektrale Bilderfassungsbaugruppe
- 20: Objektereich
- 21: abzubildendes Objekt
- 22: distales Endstück
- 24: Kamerabaugruppe
- 26a: erste Bilderfassungssensorik
- 26b: zweite Bilderfassungssensorik
- 28: Bildsensor
- 30a,b: Eingangsoptik
- 32: Strahlteiler
- 34: Blende
- 36: Aufspaltungsoptik
- 38: lichtempfindlicher Bereich
- 40: Lochblende
- 42: Blendenloch
- 44: Blendenverlagerungseinheit
- 46: Aktor
- 48: Blendenträger
- 50: Festkörpergelenk
- 52: Piezoaktor
- 54: Schlitzblende
- 56: Dove-Prisma
- 58: Drehantrieb
- 60: optische Zwischenbildverlagerungseinheit
- 62: optisches Element
- 64: Beobachtungsspalt
- 68: Kühlvorrichtung
- 70: Endoskop
- 72: Medizinisches System
- 74: Darstellungserzeugungseinheit
- 76: Bildpunkte/Bildstreifen
- 78: Darstellung
- 80: Analyseeinheit
- 82: Anzeigeeinheit
- 84: Versorgungseinheit
- 86: Lichtleiterkabel
- 88: Beleuchtungsvorrichtung
- 90: elektrisches Kabel
- 92: Steuervorrichtung
- 94: Griff
- 96a,b: Spiegel
- 98a,b: Kopplungsbereich
- 100: Endfläche
- 102: fokussierende Optik
- 103: Kollimatorlinse
- 104: Gewebestruktur
- 106: Bildbereich
- 108: Schutzglas
- 110: Bildzelle
- 114: Weißlichtbild
- 116: Lochreihe
- HL: erster Anteil einfallenden Lichts,
- SL: zweiter Anteil einfallenden Lichts, sichtbares Licht
- d: Lochreihenabstand
- d_{λ}: Aufspaltungsabstand
- ZB: Zwischenbild
- ZBA: Zwischenbildabschnitt
- ZBVP: Prisma
- LA: Längsachse
- AL: Abbildungslicht
- AP: Ausgangsposition
- VP: Verlagerungsposition

## Patentansprüche

1. Endoskopvorrichtung (10), umfassend:
einen Schaft (12), der einen proximalen Abschnitt (14) und einen distalen Abschnitt (16) aufweist; und
eine hyperspektrale Bilderfassungsbaugruppe (18), die dazu eingerichtet ist, Bilder eines Objektbereichs (20) eines abzubildenden Objekts (21) zu erfassen und hyperspektrale Bilddaten zu erzeugen, die räumliche und spektrale Information umfassen, insbesondere ferner umfassend wenigstens eine Eingangsoptik (30), durch die Abbildungslicht (AL) einkoppelbar ist, wobei die Bilderfassungsbaugruppe (18) in dem distalen Abschnitt (16), welcher vorzugsweise ein distales Endstück (22) umfasst und wobei die hyperspektrale Bilderfassungsbaugruppe (18) in dem distalen Endstück (22) angeordnet ist, angeordnet ist.

2. Endoskopvorrichtung (10) nach Anspruch 1,
ferner umfassend eine Kamerabaugruppe (24), die wenigstens eine Bilderfassungssensorik (26a) mit wenigstens einem Bildsensor (28), welcher insbesondere ein Farbbildsensor ist, umfasst, der dazu eingerichtet ist, Bilder zu erfassen und Bilddaten zu erzeugen.

3. Endoskopvorrichtung (10) nach Anspruch 2,
ferner umfassend einen Strahlteiler (32), der hinter der Eingangsoptik (30) angeordnet ist;
wobei der Strahlteiler (32) dazu eingerichtet ist, einen ersten Anteil einfallenden Lichts (HL) der hyperspektralen Bilderfassungsbaugruppe (18) zuzuführen und einen zweiten Anteil einfallenden Lichts (SL) der Kamerabaugruppe (24) zuzuführen, wobei insbesondere der Strahlteiler (32) wellenlängenselektiv ist und dazu eingerichtet ist, der Kamerabaugruppe (24) sichtbares Licht, insbesondere zumindest in einem Wellenlängenbereich von 450 nm bis 650 nm und vorzugsweise zumindest in einem Wellenlängenbereich von 420 nm bis 700 nm, zuzuführen und der hyperspektralen Bilderfassungsbaugruppe Infrarotlicht zuzuführen, insbesondere zumindest Licht mit einer Wellenlänge von über 1500 nm, bevorzugt Licht mit einer Wellenlänge von über 1000 nm und besonders bevorzugt Licht mit einer Wellenlänge von über 800 nm.

4. Endoskopvorrichtung (10) nach einem der Ansprüche 1 bis 3,
wobei die hyperspektrale Bilderfassungsbaugruppe (18) umfasst:
eine Blende (34), die dazu eingerichtet ist, das Licht (HL, SL) gemäß einem Blendenmuster auszublenden und durchzulassen;
eine Aufspaltungsoptik (36), die dazu eingerichtet ist, durch die Blende (34) durchgelassenes Licht (HL, SL) spektral aufzuspalten; und wenigstens
eine zweite Bilderfassungssensorik (26b) mit wenigstens einem Bildsensor (28), wobei die Bilderfassungssensorik (26b) einen lichtempfindlichen Bereich (38) definiert und bezüglich der Aufspaltungsoptik (36) derart angeordnet ist, dass spektral aufgespaltetes Licht auf den lichtempfindlichen Bereich (38) fällt;
wobei die Eingangsoptik (30), die Blende (34), welche insbesondere relativ zu der zweiten Bilderfassungssensorik (26b) bewegbar gelagert ist, die Aufspaltungsoptik (36) und die zweite Bilderfassungssensorik (26b) in dem distalen Abschnitt (16) angeordnet sind, wobei insbesondere die erste und/oder zweite Bilderfassungssensorik (26a,b) relativ zu dem distalen Abschnitt (16) des Schafts (12) ortsfest ist und/oder wobei die Eingangsoptik (30) und die Aufspaltungsoptik (36) relativ zu dem distalen Abschnitt (16) des Schafts (12) ortsfest sind, wobei insbesondere ein Strahlengang von dem Strahlteiler (32) zu der Bilderfassungssensorik (26a) der Kamerabaugruppe (24) und ein Strahlengang von dem Strahlteiler (32) zu der Bilderfassungssensorik (26b) der hyperspektralen Bilderfassungsbaugruppe (18) zumindest abschnittsweise parallel zueinander verlaufen.

5. Endoskopvorrichtung (10) nach einem der Ansprüche 2 bis 4,
wobei bezüglich einer Längsachse des distalen Abschnitts (16) des Schafts (12) die Kamerabaugruppe (24) und die hyperspektrale Bilderfassungsbaugruppe (18) nebeneinander und/oder hintereinander angeordnet sind.

6. Endoskopvorrichtung (10) nach einem der Ansprüche 4 bis 5,
wobei die Blende (34) als eine Lochblende (40) ausgebildet ist, die mehrere Reihen von Blendenlöchern (42), welche insbesondere bezüglich einer Querrichtung der Aufspaltungsoptik (36) und/oder des lichtempfindlichen Bereichs (38) schräg verlaufen, umfasst.

7. Endoskopvorrichtung (10) nach Anspruch 6,
wobei Reihen von Blendenlöchern (42) um einen Lochreihenabstand (d) voneinander beabstandet sind, wobei die hyperspektrale Bilderfassungsbaugruppe (18) einen auflösbaren Wellenlängenbereich definiert, und wobei die Aufspaltungsoptik (36) dazu eingerichtet ist, den auflösbaren Wellenlängenbereich entlang einer räumlichen Achse über einen Aufspaltungsabstand (λ) aufzuspalten, der höchstens so groß ist wie der Lochreihenabstand (d).

8. Endoskopvorrichtung (10) nach einem der Ansprüche 4 bis 7,
wobei die Bilderfassungsbaugruppe (18) eine Blendenverlagerungseinheit (44) umfasst, die einen Aktor (46), welcher insbesondere ein Piezoaktor (52) ist, und einen Blendenträger (48), welcher insbesondere ein Festkörpergelenk (50) aufweist, umfasst, wobei der Blendenträger (48) sowohl mit dem Aktor (46) als auch mit der Blende (34) verbunden ist, und wobei mittels des Aktors (46) die Blende (34) relativ zu der zweiten Bilderfassungssensorik (26b) bewegbar ist und wobei insbesondere der Aktor (46) dazu eingerichtet ist, eine Linearbewegung zu erzeugen, und wobei mittels des Festkörpergelenks (50) die Linearbewegung in eine Schwenkbewegung der Blende (34) wandelbar ist.

9. Endoskopvorrichtung (10) nach einem der Ansprüche 4 bis 5,
wobei die Blende (34), welche insbesondere als eine Schlitzblende (54) ausgebildet ist, um eine Rotationsachse drehbar gelagert ist, die zumindest im Wesentlichen parallel zu einer optischen Achse der Blende (34) angeordnet ist.

10. Endoskopvorrichtung (10) nach Anspruch 9,
wobei die Bilderfassungsbaugruppe (18) ein Dove-Prisma (56) umfasst, das gemeinsam mit der Blende (34) drehbar gelagert ist und/oder wobei die Bilderfassungsbaugruppe (18) einen Drehantrieb (58) umfasst, der dazu eingerichtet ist, die Blende (34) und/oder das Dove-Prisma (56) um die Rotationsachse zu drehen.

11. Endoskopvorrichtung (10) nach einem der vorhergehenden Ansprüche,
wobei die Eingangsoptik (30) dazu eingerichtet ist, ein Zwischenbild (ZB) eines abzubildenden Objekts (21) zu erzeugen, das auf die Blende (34) fällt, sodass mittels eines Beobachtungsspalts (64) der Blende (34) ein Zwischenbildabschnitt des Zwischenbilds (ZBA) auswählbar ist; und
wobei die hyperspektrale Bilderfassungseinheit (18) eine optische Zwischenbildverlagerungseinheit (60), welche insbesondere dazu eingerichtet ist, das Zwischenbild (ZB) relativ zu dem Beobachtungsspalt (64) der Blende (34) zu verlagern, und welche insbesondere ein bewegliches optisches Element (62), welches vorzugsweise zwischen der Eingangsoptik (30) und der Blende (34) angeordnet ist, umfasst, das dazu eingerichtet ist, die Position des Zwischenbilds (ZB) relativ zu dem Beobachtungsspalt (64) zu verlagern, umfasst, die dazu eingerichtet ist, das Zwischenbild (ZB) relativ zu der Blende (34) optisch zu verlagern, um unterschiedliche Zwischenbildabschnitte (ZBA) des Zwischenbilds (ZB) auszuwählen.

12. Endoskopvorrichtung (10) nach Anspruch 11,
wobei das optische Element (62) ein Prisma (ZBVP), insbesondere ein wenigstens vierseitiges Prisma (ZBVP), umfasst.

13. Endoskopvorrichtung (10) nach Anspruch 11 oder 12,
wobei die Zwischenbildverlagerungseinheit (60) einen Antrieb (58) für das optische Element (62) umfasst, der dazu eingerichtet ist, das optische Element (62) in eine vorgegebene Drehrichtung um insbesondere wenigstens 90 Grad und vorzugsweise zumindest 360 Grad oder besonders bevorzugt kontinuierlich zu drehen.

14. Endoskopvorrichtung (10) nach einem der Ansprüche 11 bis 13,
wobei die Zwischenbildverlagerungseinheit (60) einen Pendelantrieb für das optische Element (62) umfasst, der dazu eingerichtet ist, das optische Element (62) oszillatorisch zu bewegen, insbesondere gemäß einer Pendelbewegung.

15. Endoskop (70) mit einer Endoskopvorrichtung (10) nach einem der vorhergehenden Ansprüche.

16. Medizinisches System (72), umfassend:
ein Endoskop (70) gemäß Anspruch 15; und
eine Darstellungserzeugungseinheit (74), die dazu eingerichtet ist, eine Darstellung (78) für einen Benutzer zu erzeugen, die auf einem Zusammenfügen von Information unterschiedlicher Bildpunkte (76a) und/oder Bildstreifen (76b) zu einem Bildteilbereich beruht.
